# EUROPEAN PATENT APPLICATION

(11) **EP 2 679 680 A1**
(43) Date of publication of application: **01.01.2014**
(21) Application number: 12749381.5
(22) Date of filing: 19.01.2012
(51) Int. Cl.: C12N 15/09, C12N 1/21, C12P 13/12, C12N 9/88

(54) **L-CYSTEINE-PRODUCING BACTERIUM AND METHOD FOR PRODUCING L-CYSTEINE**

(30) Priority: 22.02.2011 JP 2011035289
(71) Applicant: Ajinomoto Co., Inc., Tokyo 104-8315 (JP)
(72) Inventor: TAKUMI, Kazuhiro, Kawasaki-shi Kanagawa 210-8681 (JP); NONAKA, Gen, Kawasaki-shi Kanagawa 210-8681 (JP)
(74) Representative: Strehl Schübel-Hopf & Partner
(86) International application number: PCT/JP2012/051084
(87) International publication number: WO 2012/114802

(57) **Abstract**

A novel technique for improving L-cysteine-producing ability of bacteria is developed, and thereby an L-cysteine-producing bacterium and a method for producing compounds such as L-cysteine are provided. L-cysteine, a related substance thereof, or a mixture thereof is produced by culturing a bacterium belonging to the family *Enterobacteriaceae,* which has L-cysteine-producing ability, and is modified so that L-aspartate-α-decarboxylase activity is increased, in a medium, and collecting L-cysteine, a related substance thereof, or a mixture thereof from the medium.

SEQUENCE LISTING

<110>  Ajinomoto Co., Inc.

<120>  An L-cysteine-producing bacterium and a method for producing
       L-cysteine

<130>  D290-11348

<150>  JP2011-035289
<151>  2011-02-22

<160>  60

<170>  PatentIn version 3.5

<210>  1
<211>  28
<212>  DNA
<213>  Artificial Sequence

<220>
<223>  panD(Cg)-FW

<400>  1
ccaagcttat ggcattccaa tagtcagg                                        28


<210>  2
<211>  28
<212>  DNA
<213>  Artificial Sequence

<220>
<223>  panD(Cg)-RV

<400>  2
cgaagcttgg gaaaaagacg gctctacc                                        28


<210>  3
<211>  22
<212>  DNA
<213>  Artificial Sequence

<220>
<223>  panD(Ec)-FW

<400>  3
atgattcgca cgatgctgca gg                                              22


<210>  4
<211>  23
<212>  DNA
<213>  Artificial Sequence

<220>
<223>  panD(Ec)-RV

<400>  4
tcaagcaacc tgtaccggaa tcg                                             23

<210>  5
<211>  30
<212>  DNA
<213>  Artificial Sequence

<220>
<223>  panD(Pa)-FW

<400>  5
gtaaaggctg caggtcgtat ttttggctgt                                        30


<210>  6
<211>  30
<212>  DNA
<213>  Artificial Sequence

<220>
<223>  panD(Pa)-RW

<400>  6
gttctatatc gcggtctact tcctgattca                                        30


<210>  7
<211>  381
<212>  DNA
<213>  Escherichia coli

<220>
<221>  CDS
<222>  (1)..(381)

<400>  7
atg att cgc acg atg ctg cag ggc aaa ctc cac cgc gtg aaa gtg act      48
Met Ile Arg Thr Met Leu Gln Gly Lys Leu His Arg Val Lys Val Thr
1               5                   10                  15
cat gcg gac ctg cac tat gaa ggt tct tgc gcc att gac cag gat ttt      96
His Ala Asp Leu His Tyr Glu Gly Ser Cys Ala Ile Asp Gln Asp Phe
            20                  25                  30
ctt gac gca gcc ggt att ctc gaa aac gaa gcc att gat atc tgg aat      144
Leu Asp Ala Ala Gly Ile Leu Glu Asn Glu Ala Ile Asp Ile Trp Asn
        35                  40                  45
gtc acc aac ggc aag cgt ttc tcc act tat gcc atc gcg gca gaa cgc      192
Val Thr Asn Gly Lys Arg Phe Ser Thr Tyr Ala Ile Ala Ala Glu Arg
    50                  55                  60
ggt tcg aga att att tct gtt aac ggt gcg gcg gcc cac tgc gcc agt      240
Gly Ser Arg Ile Ile Ser Val Asn Gly Ala Ala Ala His Cys Ala Ser
65                  70                  75                  80
gtc ggc gat att gtc atc atc gcc agc ttc gtt acc atg cca gat gaa      288
Val Gly Asp Ile Val Ile Ile Ala Ser Phe Val Thr Met Pro Asp Glu
                85                  90                  95
gaa gct cgc acc tgg cga ccc aac gtc gcc tat ttt gaa ggc gac aat      336
Glu Ala Arg Thr Trp Arg Pro Asn Val Ala Tyr Phe Glu Gly Asp Asn
            100                 105                 110
gaa atg aaa cgt acc gcg aaa gcg att ccg gta cag gtt gct tga          381
Glu Met Lys Arg Thr Ala Lys Ala Ile Pro Val Gln Val Ala
        115                 120                 125

<210>  8
<211>  126
<212>  PRT

<213>  Escherichia coli

<400>  8
Met Ile Arg Thr Met Leu Gln Gly Lys Leu His Arg Val Lys Val Thr
1               5                   10                  15
His Ala Asp Leu His Tyr Glu Gly Ser Cys Ala Ile Asp Gln Asp Phe
            20                  25                  30
Leu Asp Ala Ala Gly Ile Leu Glu Asn Glu Ala Ile Asp Ile Trp Asn
        35                  40                  45
Val Thr Asn Gly Lys Arg Phe Ser Thr Tyr Ala Ile Ala Ala Glu Arg
    50                  55                  60
Gly Ser Arg Ile Ile Ser Val Asn Gly Ala Ala Ala His Cys Ala Ser
65                  70                  75                  80
Val Gly Asp Ile Val Ile Ile Ala Ser Phe Val Thr Met Pro Asp Glu
                85                  90                  95
Glu Ala Arg Thr Trp Arg Pro Asn Val Ala Tyr Phe Glu Gly Asp Asn
            100                 105                 110
Glu Met Lys Arg Thr Ala Lys Ala Ile Pro Val Gln Val Ala
        115                 120                 125


<210>  9
<211>  381
<212>  DNA
<213>  Pantoea ananatis

<220>
<221>  CDS
<222>  (1)..(381)

<400>  9
atg att cgc acg gtt tta caa ggc aaa ttg cat cgc gtg aag gtg acg      48
Met Ile Arg Thr Val Leu Gln Gly Lys Leu His Arg Val Lys Val Thr
1               5                   10                  15
cag gca gac ctc aac tat gaa ggc tcc tgc gcc atc gat cag gat ttc      96
Gln Ala Asp Leu Asn Tyr Glu Gly Ser Cys Ala Ile Asp Gln Asp Phe
            20                  25                  30
ctt gat gct tct ggc atc ctg caa tac gaa gcc atc gat att tac aac      144
Leu Asp Ala Ser Gly Ile Leu Gln Tyr Glu Ala Ile Asp Ile Tyr Asn
        35                  40                  45
gtg acc aac ggt cag cgt ttc tct acc tat gct atc gcc gcc gaa cga      192
Val Thr Asn Gly Gln Arg Phe Ser Thr Tyr Ala Ile Ala Ala Glu Arg
    50                  55                  60
ggt tca aaa atc att tcg gtc aac ggc gca gcg gcg cgc tgt gcc tgt      240
Gly Ser Lys Ile Ile Ser Val Asn Gly Ala Ala Ala Arg Cys Ala Cys
65                  70                  75                  80
gaa ggc gat atc ctg att atc tgc tcc tat gtg cag gtt gaa gat gca      288
Glu Gly Asp Ile Leu Ile Ile Cys Ser Tyr Val Gln Val Glu Asp Ala
                85                  90                  95
gaa gcc cgt cag tgg aag ccc aac gtg gcc tac ttt gaa ggg gat aat      336
Glu Ala Arg Gln Trp Lys Pro Asn Val Ala Tyr Phe Glu Gly Asp Asn
            100                 105                 110
cag atg aag cgc ctg gcg aaa gcc ttg ccg gta cag ttg gcg taa          381
Gln Met Lys Arg Leu Ala Lys Ala Leu Pro Val Gln Leu Ala
        115                 120                 125


<210>  10
<211>  126
<212>  PRT
<213>  Pantoea ananatis

<400>  10

Met Ile Arg Thr Val Leu Gln Gly Lys Leu His Arg Val Lys Val Thr
1               5                   10                  15
Gln Ala Asp Leu Asn Tyr Glu Gly Ser Cys Ala Ile Asp Gln Asp Phe
            20                  25                  30
Leu Asp Ala Ser Gly Ile Leu Gln Tyr Glu Ala Ile Asp Ile Tyr Asn
        35                  40                  45
Val Thr Asn Gly Gln Arg Phe Ser Thr Tyr Ala Ile Ala Ala Glu Arg
    50                  55                  60
Gly Ser Lys Ile Ile Ser Val Asn Gly Ala Ala Ala Arg Cys Ala Cys
65                  70                  75                  80
Glu Gly Asp Ile Leu Ile Ile Cys Ser Tyr Val Gln Val Glu Asp Ala
                85                  90                  95
Glu Ala Arg Gln Trp Lys Pro Asn Val Ala Tyr Phe Glu Gly Asp Asn
            100                 105                 110
Gln Met Lys Arg Leu Ala Lys Ala Leu Pro Val Gln Leu Ala
        115                 120                 125

<210>  11
<211>  411
<212>  DNA
<213>  Corynebacterium glutamicum

<220>
<221>  CDS
<222>  (1)..(411)

<400>  11
atg ctg cgc acc atc ctc gga agt aag att cac cga gcc act gtc act        48
Met Leu Arg Thr Ile Leu Gly Ser Lys Ile His Arg Ala Thr Val Thr
1               5                   10                  15
caa gct gat cta gat tat gtt ggc tct gta acc atc gac gcc gac ctg        96
Gln Ala Asp Leu Asp Tyr Val Gly Ser Val Thr Ile Asp Ala Asp Leu
            20                  25                  30
gtt cac gcc gcc gga ttg atc gaa ggc gaa aaa gtt gcc atc gta gac       144
Val His Ala Ala Gly Leu Ile Glu Gly Glu Lys Val Ala Ile Val Asp
        35                  40                  45
atc acc aac ggc gct cgt ctg gaa act tat gtc att gtg ggc gac gcc       192
Ile Thr Asn Gly Ala Arg Leu Glu Thr Tyr Val Ile Val Gly Asp Ala
    50                  55                  60
gga acg ggc aat att tgc atc aat ggt gcc gct gca cac ctt att aat       240
Gly Thr Gly Asn Ile Cys Ile Asn Gly Ala Ala Ala His Leu Ile Asn
65                  70                  75                  80
cct ggc gat ctt gtg atc atc atg agc tac ctt cag gca act gat gcg       288
Pro Gly Asp Leu Val Ile Ile Met Ser Tyr Leu Gln Ala Thr Asp Ala
                85                  90                  95
gaa gcc aag gcg tat gag cca aag att gtg cac gtg gac gcc gac aac       336
Glu Ala Lys Ala Tyr Glu Pro Lys Ile Val His Val Asp Ala Asp Asn
            100                 105                 110
cgc atc gtt gcg ctc ggc aac gat ctt gcg gaa gca cta cct gga tcc       384
Arg Ile Val Ala Leu Gly Asn Asp Leu Ala Glu Ala Leu Pro Gly Ser
        115                 120                 125
ggg ctt ttg acg tcg aga agc att tag                                   411
Gly Leu Leu Thr Ser Arg Ser Ile
    130                 135

<210>  12
<211>  136
<212>  PRT
<213>  Corynebacterium glutamicum

<400>  12

Met Leu Arg Thr Ile Leu Gly Ser Lys Ile His Arg Ala Thr Val Thr
1               5                   10                  15
Gln Ala Asp Leu Asp Tyr Val Gly Ser Val Thr Ile Asp Ala Asp Leu
            20                  25                  30
Val His Ala Ala Gly Leu Ile Glu Gly Glu Lys Val Ala Ile Val Asp
        35                  40                  45
Ile Thr Asn Gly Ala Arg Leu Glu Thr Tyr Val Ile Val Gly Asp Ala
    50                  55                  60
Gly Thr Gly Asn Ile Cys Ile Asn Gly Ala Ala Ala His Leu Ile Asn
65                  70                  75                  80
Pro Gly Asp Leu Val Ile Ile Met Ser Tyr Leu Gln Ala Thr Asp Ala
                85                  90                  95
Glu Ala Lys Ala Tyr Glu Pro Lys Ile Val His Val Asp Ala Asp Asn
            100                 105                 110
Arg Ile Val Ala Leu Gly Asn Asp Leu Ala Glu Ala Leu Pro Gly Ser
        115                 120                 125
Gly Leu Leu Thr Ser Arg Ser Ile
    130                 135

<210>  13
<211>  381
<212>  DNA
<213>  Bacillus subtilis

<220>
<221>  CDS
<222>  (1)..(381)

<400>  13
atg tat cga aca atg atg agc ggc aaa ctt cac agg gca act gtt acg      48
Met Tyr Arg Thr Met Met Ser Gly Lys Leu His Arg Ala Thr Val Thr
1               5                   10                  15
gaa gca aac ctg aac tat gtg gga agc att aca att gat gaa gat ctc      96
Glu Ala Asn Leu Asn Tyr Val Gly Ser Ile Thr Ile Asp Glu Asp Leu
            20                  25                  30
att gat gct gtg gga atg ctt cct aat gaa aaa gta caa att gtg aat      144
Ile Asp Ala Val Gly Met Leu Pro Asn Glu Lys Val Gln Ile Val Asn
        35                  40                  45
aat aat aat gga gca cgt ctt gaa acg tat att att cct ggt aaa cgg      192
Asn Asn Asn Gly Ala Arg Leu Glu Thr Tyr Ile Ile Pro Gly Lys Arg
    50                  55                  60
gga agc ggc gtc ata tgc tta aac ggt gca gcc gca cgc ctt gtg cag      240
Gly Ser Gly Val Ile Cys Leu Asn Gly Ala Ala Ala Arg Leu Val Gln
65                  70                  75                  80
gaa gga gat aag gtc att att att tcc tac aaa atg atg tct gat caa      288
Glu Gly Asp Lys Val Ile Ile Ile Ser Tyr Lys Met Met Ser Asp Gln
                85                  90                  95
gaa gcg gca agc cat gag ccg aaa gtg gct gtt ctg aat gat caa aac      336
Glu Ala Ala Ser His Glu Pro Lys Val Ala Val Leu Asn Asp Gln Asn
            100                 105                 110
aaa att gaa caa atg ctg ggg aac gaa cca gcc cgt aca att ttg          381
Lys Ile Glu Gln Met Leu Gly Asn Glu Pro Ala Arg Thr Ile Leu
        115                 120                 125

<210>  14
<211>  127
<212>  PRT
<213>  Bacillus subtilis

<400>  14
Met Tyr Arg Thr Met Met Ser Gly Lys Leu His Arg Ala Thr Val Thr

1               5                   10                  15
Glu Ala Asn Leu Asn Tyr Val Gly Ser Ile Thr Ile Asp Glu Asp Leu
            20                  25                  30
Ile Asp Ala Val Gly Met Leu Pro Asn Glu Lys Val Gln Ile Val Asn
        35                  40                  45
Asn Asn Asn Gly Ala Arg Leu Glu Thr Tyr Ile Ile Pro Gly Lys Arg
    50                  55                  60
Gly Ser Gly Val Ile Cys Leu Asn Gly Ala Ala Ala Arg Leu Val Gln
65                  70                  75                  80
Glu Gly Asp Lys Val Ile Ile Ile Ser Tyr Lys Met Met Ser Asp Gln
                85                  90                  95
Glu Ala Ala Ser His Glu Pro Lys Val Ala Val Leu Asn Asp Gln Asn
            100                 105                 110
Lys Ile Glu Gln Met Leu Gly Asn Glu Pro Ala Arg Thr Ile Leu
        115                 120                 125

<210>  15
<211>  432
<212>  DNA
<213>  Arthrobacter aurescens

<220>
<221>  CDS
<222>  (1)..(432)

<400>  15
atg atc cgc aca atg ttc aag tcc aag att cac cgc gct acg gtg acc        48
Met Ile Arg Thr Met Phe Lys Ser Lys Ile His Arg Ala Thr Val Thr
1               5                   10                  15
cac gcc gac ctc cac tac gtc ggt tcg gtc act gtc gac ctg gac ctc        96
His Ala Asp Leu His Tyr Val Gly Ser Val Thr Val Asp Leu Asp Leu
            20                  25                  30
ctg gac gcg gcc gac atc ctt ccc ggc gag ctc gtc tcc att gtt gac       144
Leu Asp Ala Ala Asp Ile Leu Pro Gly Glu Leu Val Ser Ile Val Asp
        35                  40                  45
gtg acc aac ggc gcc cgg ctg gag acg tac acc att gcc ggc gag cgc       192
Val Thr Asn Gly Ala Arg Leu Glu Thr Tyr Thr Ile Ala Gly Glu Arg
    50                  55                  60
ggt tca ggc gtg atc ggg atc aac ggc gcg gcc gcc cat ctg gta cat       240
Gly Ser Gly Val Ile Gly Ile Asn Gly Ala Ala Ala His Leu Val His
65                  70                  75                  80
gtg gat gac acg gtc atc ctc att acc tac gca gaa atg acc acg gaa       288
Val Asp Asp Thr Val Ile Leu Ile Thr Tyr Ala Glu Met Thr Thr Glu
                85                  90                  95
gag gcc cgg aca tac gag ccc agg gtg gtc cac gtg ggc aag gac aac       336
Glu Ala Arg Thr Tyr Glu Pro Arg Val Val His Val Gly Lys Asp Asn
            100                 105                 110
aag atc ctg cag ctc ggc aac gac ccc gct gag ggc cac act ccc ggg       384
Lys Ile Leu Gln Leu Gly Asn Asp Pro Ala Glu Gly His Thr Pro Gly
        115                 120                 125
ctc atg cgc cct ccg cac gca ctc agt aac gcg gcg cac ctc agc taa       432
Leu Met Arg Pro Pro His Ala Leu Ser Asn Ala Ala His Leu Ser
    130                 135                 140

<210>  16
<211>  143
<212>  PRT
<213>  Arthrobacter aurescens

<400>  16
Met Ile Arg Thr Met Phe Lys Ser Lys Ile His Arg Ala Thr Val Thr

1               5                   10                  15
His Ala Asp Leu His Tyr Val Gly Ser Val Thr Val Asp Leu Asp Leu
            20                  25                  30
Leu Asp Ala Ala Asp Ile Leu Pro Gly Glu Leu Val Ser Ile Val Asp
        35                  40                  45
Val Thr Asn Gly Ala Arg Leu Glu Thr Tyr Thr Ile Ala Gly Glu Arg
    50                  55                  60
Gly Ser Gly Val Ile Gly Ile Asn Gly Ala Ala Ala His Leu Val His
65                  70                  75                  80
Val Asp Asp Thr Val Ile Leu Ile Thr Tyr Ala Glu Met Thr Thr Glu
                85                  90                  95
Glu Ala Arg Thr Tyr Glu Pro Arg Val Val His Val Gly Lys Asp Asn
            100                 105                 110
Lys Ile Leu Gln Leu Gly Asn Asp Pro Ala Glu Gly His Thr Pro Gly
        115                 120                 125
Leu Met Arg Pro Pro His Ala Leu Ser Asn Ala Ala His Leu Ser
    130                 135                 140


<210>  17
<211>  354
<212>  DNA
<213>  Bacteroides fragilis

<220>
<221>  CDS
<222>  (1)..(354)

<400>  17
atg atg att gaa gtg ttg aag tca aaa att cat tgt gcc cgt gtc acg      48
Met Met Ile Glu Val Leu Lys Ser Lys Ile His Cys Ala Arg Val Thr
1               5                   10                  15
gaa gcc aat ctg aat tat atg ggt agt att acg att gat gag aat cta      96
Glu Ala Asn Leu Asn Tyr Met Gly Ser Ile Thr Ile Asp Glu Asn Leu
            20                  25                  30
ttg gat gca gct aat atg att gcc ggt gag aag gtg tac att gcc gac      144
Leu Asp Ala Ala Asn Met Ile Ala Gly Glu Lys Val Tyr Ile Ala Asp
        35                  40                  45
aat aat aac ggc gag cgt ttt gag acc tac att atc aaa ggt gaa cgc      192
Asn Asn Asn Gly Glu Arg Phe Glu Thr Tyr Ile Ile Lys Gly Glu Arg
    50                  55                  60
gga tca ggt aaa atc tgc ttg aat ggc gct gcg gcc cgt aaa gta caa      240
Gly Ser Gly Lys Ile Cys Leu Asn Gly Ala Ala Ala Arg Lys Val Gln
65                  70                  75                  80
ccg gat gat att gtg atc att atg tcg tat gcg ttg atg gat ttt gag      288
Pro Asp Asp Ile Val Ile Ile Met Ser Tyr Ala Leu Met Asp Phe Glu
                85                  90                  95
gaa gct aag tcg ttt aag ccg act gtt att ttt ccg gat ccg gct aca      336
Glu Ala Lys Ser Phe Lys Pro Thr Val Ile Phe Pro Asp Pro Ala Thr
            100                 105                 110
aat agt gta gtg aag taa                                              354
Asn Ser Val Val Lys
        115


<210>  18
<211>  117
<212>  PRT
<213>  Bacteroides fragilis

<400>  18
Met Met Ile Glu Val Leu Lys Ser Lys Ile His Cys Ala Arg Val Thr
1               5                   10                  15

Glu Ala Asn Leu Asn Tyr Met Gly Ser Ile Thr Ile Asp Glu Asn Leu
            20                  25                  30
Leu Asp Ala Ala Asn Met Ile Ala Gly Glu Lys Val Tyr Ile Ala Asp
        35                  40                  45
Asn Asn Asn Gly Glu Arg Phe Glu Thr Tyr Ile Ile Lys Gly Glu Arg
    50                  55                  60
Gly Ser Gly Lys Ile Cys Leu Asn Gly Ala Ala Ala Arg Lys Val Gln
65                  70                  75                  80
Pro Asp Asp Ile Val Ile Ile Met Ser Tyr Ala Leu Met Asp Phe Glu
                85                  90                  95
Glu Ala Lys Ser Phe Lys Pro Thr Val Ile Phe Pro Asp Pro Ala Thr
            100                 105                 110
Asn Ser Val Val Lys
        115

<210> 19
<211> 351
<212> DNA
<213> Bacteroides vulgatus

<220>
<221> CDS
<222> (1)..(351)

<400> 19
atg atg att gag gta tta aag tcg aaa ctg cat tgt gtc cgt gta acg 48
Met Met Ile Glu Val Leu Lys Ser Lys Leu His Cys Val Arg Val Thr
1               5                   10                  15
gaa gcc aat ttg aat tac atg ggt agt att acg att gac gag gac ttg 96
Glu Ala Asn Leu Asn Tyr Met Gly Ser Ile Thr Ile Asp Glu Asp Leu
            20                  25                  30
atg gat gcc gcc aac atg att gcc ggc gag aaa gtg cac att gtg gat 144
Met Asp Ala Ala Asn Met Ile Ala Gly Glu Lys Val His Ile Val Asp
        35                  40                  45
aat aat aac ggc gag agg ttt gaa act tat att att aag ggt gag cgc 192
Asn Asn Asn Gly Glu Arg Phe Glu Thr Tyr Ile Ile Lys Gly Glu Arg
    50                  55                  60
ggt tcg gga tgt att tgt ctg aat ggg gct gct gcc cgg aaa gta cag 240
Gly Ser Gly Cys Ile Cys Leu Asn Gly Ala Ala Ala Arg Lys Val Gln
65                  70                  75                  80
gtg ggt gac att gtg att atc atg tca tac gcc atg atg gat ttt gag 288
Val Gly Asp Ile Val Ile Ile Met Ser Tyr Ala Met Met Asp Phe Glu
                85                  90                  95
gaa gct aaa tcg ttc aag ccg acg gtg gtt ttc cct gat tcg gcg atg 336
Glu Ala Lys Ser Phe Lys Pro Thr Val Val Phe Pro Asp Ser Ala Met
            100                 105                 110
aac aag att gta taa 351
Asn Lys Ile Val
        115

<210> 20
<211> 116
<212> PRT
<213> Bacteroides vulgatus

<400> 20
Met Met Ile Glu Val Leu Lys Ser Lys Leu His Cys Val Arg Val Thr
1               5                   10                  15
Glu Ala Asn Leu Asn Tyr Met Gly Ser Ile Thr Ile Asp Glu Asp Leu
            20                  25                  30
Met Asp Ala Ala Asn Met Ile Ala Gly Glu Lys Val His Ile Val Asp

35                  40                  45
Asn Asn Asn Gly Glu Arg Phe Glu Thr Tyr Ile Ile Lys Gly Glu Arg
    50                  55                  60
Gly Ser Gly Cys Ile Cys Leu Asn Gly Ala Ala Ala Arg Lys Val Gln
65                  70                  75                  80
Val Gly Asp Ile Val Ile Ile Met Ser Tyr Ala Met Met Asp Phe Glu
                85                  90                  95
Glu Ala Lys Ser Phe Lys Pro Thr Val Val Phe Pro Asp Ser Ala Met
            100                 105                 110
Asn Lys Ile Val
        115

<210>  21
<211>  411
<212>  DNA
<213>  Brevibacterium lactofermentum

<220>
<221>  CDS
<222>  (1)..(411)

<400>  21
atg ctg cgc acc atc ctc gga agt aag att cac cga gcc act gtc act      48
Met Leu Arg Thr Ile Leu Gly Ser Lys Ile His Arg Ala Thr Val Thr
1               5                   10                  15
caa gct gat cta gat tat gtt ggc tct gta acc atc gac gcc gac ctg      96
Gln Ala Asp Leu Asp Tyr Val Gly Ser Val Thr Ile Asp Ala Asp Leu
            20                  25                  30
gtt cac gcc gcc gga ttg atc gaa ggc gaa aaa gtt gcc atc gta gac     144
Val His Ala Ala Gly Leu Ile Glu Gly Glu Lys Val Ala Ile Val Asp
        35                  40                  45
atc acc aac ggc gct cgt ctg gaa act tat gtc att gtg ggc gac gcc     192
Ile Thr Asn Gly Ala Arg Leu Glu Thr Tyr Val Ile Val Gly Asp Ala
    50                  55                  60
aga acg ggc aat att tgc atc aat ggt gcc gct gca cac ctt att aat     240
Arg Thr Gly Asn Ile Cys Ile Asn Gly Ala Ala Ala His Leu Ile Asn
65                  70                  75                  80
cct ggc gat ctt gtg atc atc atg agc tac ctt cag gcg act gat gcg     288
Pro Gly Asp Leu Val Ile Ile Met Ser Tyr Leu Gln Ala Thr Asp Ala
                85                  90                  95
gaa gcc aag gcg tat gag cca aag att gtg cac gtg gac gcc gac aac     336
Glu Ala Lys Ala Tyr Glu Pro Lys Ile Val His Val Asp Ala Asp Asn
            100                 105                 110
cgc atc gtt gcg ctc ggc aac gat ctt gcg gaa gca cta cct gga tcc     384
Arg Ile Val Ala Leu Gly Asn Asp Leu Ala Glu Ala Leu Pro Gly Ser
        115                 120                 125
ggg ctt ttg acg tcg aga agc att tag                                 411
Gly Leu Leu Thr Ser Arg Ser Ile
    130                 135

<210>  22
<211>  136
<212>  PRT
<213>  Brevibacterium lactofermentum

<400>  22
Met Leu Arg Thr Ile Leu Gly Ser Lys Ile His Arg Ala Thr Val Thr
1               5                   10                  15
Gln Ala Asp Leu Asp Tyr Val Gly Ser Val Thr Ile Asp Ala Asp Leu
            20                  25                  30
Val His Ala Ala Gly Leu Ile Glu Gly Glu Lys Val Ala Ile Val Asp

35                  40                  45
Ile Thr Asn Gly Ala Arg Leu Glu Thr Tyr Val Ile Val Gly Asp Ala
    50                  55                  60
Arg Thr Gly Asn Ile Cys Ile Asn Gly Ala Ala Ala His Leu Ile Asn
65                  70                  75                  80
Pro Gly Asp Leu Val Ile Ile Met Ser Tyr Leu Gln Ala Thr Asp Ala
                85                  90                  95
Glu Ala Lys Ala Tyr Glu Pro Lys Ile Val His Val Asp Ala Asp Asn
            100                 105                 110
Arg Ile Val Ala Leu Gly Asn Asp Leu Ala Glu Ala Leu Pro Gly Ser
        115                 120                 125
Gly Leu Leu Thr Ser Arg Ser Ile
    130                 135

<210>  23
<211>  387
<212>  DNA
<213>  Chlorobium tepidum

<220>
<221>  CDS
<222>  (1)..(387)

<400>  23
atg aaa tta cac atg ctc aaa tcc aag atc cac aac gcc att gtc acc      48
Met Lys Leu His Met Leu Lys Ser Lys Ile His Asn Ala Ile Val Thr
1               5                   10                  15
agt gga gac ctg gaa tac gaa gga agc atc acc atc gac aag gag ctg      96
Ser Gly Asp Leu Glu Tyr Glu Gly Ser Ile Thr Ile Asp Lys Glu Leu
            20                  25                  30
ctt gac atg gcc gat atg atc gcc aat gaa aag gtg ctg gtt gtc aat      144
Leu Asp Met Ala Asp Met Ile Ala Asn Glu Lys Val Leu Val Val Asn
        35                  40                  45
aac aac aac ggt gag cga ttc gag act tac atc atc gag gga aca cgc      192
Asn Asn Asn Gly Glu Arg Phe Glu Thr Tyr Ile Ile Glu Gly Thr Arg
    50                  55                  60
gga ctg cgc gaa atc cag ctc aat ggc gcg gcg gcc cgt tgc gcg ctg      240
Gly Leu Arg Glu Ile Gln Leu Asn Gly Ala Ala Ala Arg Cys Ala Leu
65                  70                  75                  80
ccc ggc gac gag atc atc atc atg acc ttc gcc gaa atg gaa ccg gaa      288
Pro Gly Asp Glu Ile Ile Ile Met Thr Phe Ala Glu Met Glu Pro Glu
                85                  90                  95
gag gcg cgc aac tgg aaa ccg atg att gtc atc gtt gac cgg atg aac      336
Glu Ala Arg Asn Trp Lys Pro Met Ile Val Ile Val Asp Arg Met Asn
            100                 105                 110
aat ccg aag cga cgc cac cgg gta ggt tcc gaa gac gaa tat ctt ggc      384
Asn Pro Lys Arg Arg His Arg Val Gly Ser Glu Asp Glu Tyr Leu Gly
        115                 120                 125
tga                                                                  387

<210>  24
<211>  128
<212>  PRT
<213>  Chlorobium tepidum

<400>  24
Met Lys Leu His Met Leu Lys Ser Lys Ile His Asn Ala Ile Val Thr
1               5                   10                  15
Ser Gly Asp Leu Glu Tyr Glu Gly Ser Ile Thr Ile Asp Lys Glu Leu
            20                  25                  30
Leu Asp Met Ala Asp Met Ile Ala Asn Glu Lys Val Leu Val Val Asn

35                  40                  45
Asn Asn Asn Gly Glu Arg Phe Glu Thr Tyr Ile Ile Glu Gly Thr Arg
    50                  55                  60
Gly Leu Arg Glu Ile Gln Leu Asn Gly Ala Ala Ala Arg Cys Ala Leu
65                  70                  75                  80
Pro Gly Asp Glu Ile Ile Ile Met Thr Phe Ala Glu Met Glu Pro Glu
                85                  90                  95
Glu Ala Arg Asn Trp Lys Pro Met Ile Val Ile Val Asp Arg Met Asn
            100                 105                 110
Asn Pro Lys Arg Arg His Arg Val Gly Ser Glu Asp Glu Tyr Leu Gly
        115                 120                 125

<210>  25
<211>  381
<212>  DNA
<213>  Citrobacter koseri

<220>
<221>  CDS
<222>  (1)..(381)

<400>  25
atg att cgc act atg ctg cag ggc aaa ctc cac cgc gtg aaa gtg act        48
Met Ile Arg Thr Met Leu Gln Gly Lys Leu His Arg Val Lys Val Thr
1               5                   10                  15
cag gcg gac ctg cac tac gaa ggt tcc tgc gcc att gat caa gat ttt        96
Gln Ala Asp Leu His Tyr Glu Gly Ser Cys Ala Ile Asp Gln Asp Phe
            20                  25                  30
ctg gat gca gcc ggt att ctt gaa aac gaa gcc att gat atc tgg aac        144
Leu Asp Ala Ala Gly Ile Leu Glu Asn Glu Ala Ile Asp Ile Trp Asn
        35                  40                  45
gtg act aac ggc aag cgc ttc tca acc tat gcg att gcc gcc gag cgt        192
Val Thr Asn Gly Lys Arg Phe Ser Thr Tyr Ala Ile Ala Ala Glu Arg
    50                  55                  60
ggt tcc aga atc atc tcc gtg aac ggc gca gcg gcg cac tgc gct gcg        240
Gly Ser Arg Ile Ile Ser Val Asn Gly Ala Ala Ala His Cys Ala Ala
65                  70                  75                  80
gtg ggc gat atc gtg atc atc gca agt ttc gtt acc atg tca gac gaa        288
Val Gly Asp Ile Val Ile Ile Ala Ser Phe Val Thr Met Ser Asp Glu
                85                  90                  95
gaa gcg cgt cgc tgg cag ccc aac gtc gcc tat ttt gaa ggc gat aat        336
Glu Ala Arg Arg Trp Gln Pro Asn Val Ala Tyr Phe Glu Gly Asp Asn
            100                 105                 110
gaa atg aag cgc acc gcg aaa gcg atc ccg gta cag gtt gcc tga            381
Glu Met Lys Arg Thr Ala Lys Ala Ile Pro Val Gln Val Ala
        115                 120                 125

<210>  26
<211>  126
<212>  PRT
<213>  Citrobacter koseri

<400>  26
Met Ile Arg Thr Met Leu Gln Gly Lys Leu His Arg Val Lys Val Thr
1               5                   10                  15
Gln Ala Asp Leu His Tyr Glu Gly Ser Cys Ala Ile Asp Gln Asp Phe
            20                  25                  30
Leu Asp Ala Ala Gly Ile Leu Glu Asn Glu Ala Ile Asp Ile Trp Asn
        35                  40                  45
Val Thr Asn Gly Lys Arg Phe Ser Thr Tyr Ala Ile Ala Ala Glu Arg
    50                  55                  60

Gly Ser Arg Ile Ile Ser Val Asn Gly Ala Ala Ala His Cys Ala Ala
65                  70                  75                  80
Val Gly Asp Ile Val Ile Ile Ala Ser Phe Val Thr Met Ser Asp Glu
                85                  90                  95
Glu Ala Arg Arg Trp Gln Pro Asn Val Ala Tyr Phe Glu Gly Asp Asn
            100                 105                 110
Glu Met Lys Arg Thr Ala Lys Ala Ile Pro Val Gln Val Ala
        115                 120                 125

<210>  27
<211>  417
<212>  DNA
<213>  Clavibacter michiganensis

<220>
<221>  CDS
<222>  (1)..(417)

<400>  27
atg ctg cgc acc atg atg acc gcg aag atc cac cgc gcc acg gtg acg      48
Met Leu Arg Thr Met Met Thr Ala Lys Ile His Arg Ala Thr Val Thr
1               5                   10                  15
cac gcc gac ctg cac tac gtc ggc tcc gtc acg gtc gac cgc gac ctg      96
His Ala Asp Leu His Tyr Val Gly Ser Val Thr Val Asp Arg Asp Leu
            20                  25                  30
ctc gac gcc gct gac atc ctc gtg ggc gag cgg gtc agc atc gtc gac      144
Leu Asp Ala Ala Asp Ile Leu Val Gly Glu Arg Val Ser Ile Val Asp
        35                  40                  45
gtc acc aac ggc gcc cgg ctc gac acc tac acg atc gcg ggc gag cgc      192
Val Thr Asn Gly Ala Arg Leu Asp Thr Tyr Thr Ile Ala Gly Glu Arg
    50                  55                  60
ggc agc ggc gtg ctc ggg atc aac ggc gcg gcc gcg cac ctc gtg gac      240
Gly Ser Gly Val Leu Gly Ile Asn Gly Ala Ala Ala His Leu Val Asp
65                  70                  75                  80
gtc ggc gac gtg gtg atc ctc atc gcg tac ggc cag atg acg acc gag      288
Val Gly Asp Val Val Ile Leu Ile Ala Tyr Gly Gln Met Thr Thr Glu
                85                  90                  95
gag gcc cgc gcg ctc gag ccc cgc gtg gtg cac gtc gac gtc ggc aac      336
Glu Ala Arg Ala Leu Glu Pro Arg Val Val His Val Asp Val Gly Asn
            100                 105                 110
cgc atc cgc gcc gtg gac gcc gac ccc acc gcg ccg ccc gcg ccc ggg      384
Arg Ile Arg Ala Val Asp Ala Asp Pro Thr Ala Pro Pro Ala Pro Gly
        115                 120                 125
ctc gag cgc tcc ccg ctg gcc gag ccg gtc tga                          417
Leu Glu Arg Ser Pro Leu Ala Glu Pro Val
    130                 135

<210>  28
<211>  138
<212>  PRT
<213>  Clavibacter michiganensis

<400>  28
Met Leu Arg Thr Met Met Thr Ala Lys Ile His Arg Ala Thr Val Thr
1               5                   10                  15
His Ala Asp Leu His Tyr Val Gly Ser Val Thr Val Asp Arg Asp Leu
            20                  25                  30
Leu Asp Ala Ala Asp Ile Leu Val Gly Glu Arg Val Ser Ile Val Asp
        35                  40                  45
Val Thr Asn Gly Ala Arg Leu Asp Thr Tyr Thr Ile Ala Gly Glu Arg
    50                  55                  60

Gly Ser Gly Val Leu Gly Ile Asn Gly Ala Ala Ala His Leu Val Asp
65                  70                  75                  80
Val Gly Asp Val Val Ile Leu Ile Ala Tyr Gly Gln Met Thr Thr Glu
                85                  90                  95
Glu Ala Arg Ala Leu Glu Pro Arg Val Val His Val Asp Val Gly Asn
            100                 105                 110
Arg Ile Arg Ala Val Asp Ala Asp Pro Thr Ala Pro Pro Ala Pro Gly
        115                 120                 125
Leu Glu Arg Ser Pro Leu Ala Glu Pro Val
    130                 135

<210>  29
<211>  417
<212>  DNA
<213>  Corynebacterium jeikeium

<220>
<221>  CDS
<222>  (1)..(417)

<400>  29
gtg ttg cga aca atg ctg aaa tca aag atc cac cgt gcg acg gtc acg        48
Met Leu Arg Thr Met Leu Lys Ser Lys Ile His Arg Ala Thr Val Thr
1               5                   10                  15
cag gcc gac ttg cac tac gtc ggc tcg tgc acc atc gac gct gat ctg        96
Gln Ala Asp Leu His Tyr Val Gly Ser Cys Thr Ile Asp Ala Asp Leu
            20                  25                  30
atg gaa gct gcc gac atc ctc gag ggc gag cag atc gat atc gtc gat       144
Met Glu Ala Ala Asp Ile Leu Glu Gly Glu Gln Ile Asp Ile Val Asp
        35                  40                  45
atc gat aac ggc aat cgc ctg acg acc tac gcc atc acc ggc gac cgg       192
Ile Asp Asn Gly Asn Arg Leu Thr Thr Tyr Ala Ile Thr Gly Asp Arg
    50                  55                  60
ggt acc ggc gtg atc ggc atc aac ggt gcc gcc gcg cgc ctg atc agc       240
Gly Thr Gly Val Ile Gly Ile Asn Gly Ala Ala Ala Arg Leu Ile Ser
65                  70                  75                  80
ccc ggc gac ctc gtc atc atc atc ggc tat gcc cag tac agc gag gaa       288
Pro Gly Asp Leu Val Ile Ile Ile Gly Tyr Ala Gln Tyr Ser Glu Glu
                85                  90                  95
gac ctg aag aac tac gcc tcg cgc gtg gtc ttc gtg gac gga aac aac       336
Asp Leu Lys Asn Tyr Ala Ser Arg Val Val Phe Val Asp Gly Asn Asn
            100                 105                 110
aag cag gtg gag ctc ggc ggc gac ccg gcc cag gtg ccg gac ggc tcg       384
Lys Gln Val Glu Leu Gly Gly Asp Pro Ala Gln Val Pro Asp Gly Ser
        115                 120                 125
ggg cta aag aac ccg cgc cac ccg gag gcc tag                           417
Gly Leu Lys Asn Pro Arg His Pro Glu Ala
    130                 135

<210>  30
<211>  138
<212>  PRT
<213>  Corynebacterium jeikeium

<400>  30
Met Leu Arg Thr Met Leu Lys Ser Lys Ile His Arg Ala Thr Val Thr
1               5                   10                  15
Gln Ala Asp Leu His Tyr Val Gly Ser Cys Thr Ile Asp Ala Asp Leu
            20                  25                  30
Met Glu Ala Ala Asp Ile Leu Glu Gly Glu Gln Ile Asp Ile Val Asp
        35                  40                  45

Ile Asp Asn Gly Asn Arg Leu Thr Thr Tyr Ala Ile Thr Gly Asp Arg
    50                  55                  60
Gly Thr Gly Val Ile Gly Ile Asn Gly Ala Ala Ala Arg Leu Ile Ser
65                  70                  75                  80
Pro Gly Asp Leu Val Ile Ile Ile Gly Tyr Ala Gln Tyr Ser Glu Glu
                85                  90                  95
Asp Leu Lys Asn Tyr Ala Ser Arg Val Val Phe Val Asp Gly Asn Asn
            100                 105                 110
Lys Gln Val Glu Leu Gly Gly Asp Pro Ala Gln Val Pro Asp Gly Ser
        115                 120                 125
Gly Leu Lys Asn Pro Arg His Pro Glu Ala
    130                 135

<210>  31
<211>  348
<212>  DNA
<213>  Cytophaga hutchinsonii

<220>
<221>  CDS
<222>  (1)..(348)

<400>  31
atg aat att gaa gta cta aaa tcg aaa atc cat cgc gtt aag gtt aca      48
Met Asn Ile Glu Val Leu Lys Ser Lys Ile His Arg Val Lys Val Thr
1               5                   10                  15
cag gcg gaa tta cat tat gtt gga agc att aca att gac gaa gcg ttg      96
Gln Ala Glu Leu His Tyr Val Gly Ser Ile Thr Ile Asp Glu Ala Leu
            20                  25                  30
atg gag gcg tcc aat att att gaa aac gaa aaa gtt cag atc gtt aac      144
Met Glu Ala Ser Asn Ile Ile Glu Asn Glu Lys Val Gln Ile Val Asn
        35                  40                  45
atc aac aac ggt gaa cgt ttt gaa aca tac gtg atc aaa ggc gaa aaa      192
Ile Asn Asn Gly Glu Arg Phe Glu Thr Tyr Val Ile Lys Gly Glu Lys
    50                  55                  60
aac acc ggt gta att tgt tta aac ggc ccg gca gcc cgt aag gta gct      240
Asn Thr Gly Val Ile Cys Leu Asn Gly Pro Ala Ala Arg Lys Val Ala
65                  70                  75                  80
gtg ggt gat atc gta atc gtt atc gca tac gcg tcc atg gat ttt gaa      288
Val Gly Asp Ile Val Ile Val Ile Ala Tyr Ala Ser Met Asp Phe Glu
                85                  90                  95
gag gca aaa aaa tgg aaa cct acc ttg att ttt ccg gat gcc aat aac      336
Glu Ala Lys Lys Trp Lys Pro Thr Leu Ile Phe Pro Asp Ala Asn Asn
            100                 105                 110
aga ctt att taa                                                      348
Arg Leu Ile
        115

<210>  32
<211>  115
<212>  PRT
<213>  Cytophaga hutchinsonii

<400>  32
Met Asn Ile Glu Val Leu Lys Ser Lys Ile His Arg Val Lys Val Thr
1               5                   10                  15
Gln Ala Glu Leu His Tyr Val Gly Ser Ile Thr Ile Asp Glu Ala Leu
            20                  25                  30
Met Glu Ala Ser Asn Ile Ile Glu Asn Glu Lys Val Gln Ile Val Asn
        35                  40                  45
Ile Asn Asn Gly Glu Arg Phe Glu Thr Tyr Val Ile Lys Gly Glu Lys

50                  55                  60
Asn Thr Gly Val Ile Cys Leu Asn Gly Pro Ala Ala Arg Lys Val Ala
65                  70                  75                  80
Val Gly Asp Ile Val Ile Val Ile Ala Tyr Ala Ser Met Asp Phe Glu
                85                  90                  95
Glu Ala Lys Lys Trp Lys Pro Thr Leu Ile Phe Pro Asp Ala Asn Asn
            100                 105                 110
Arg Leu Ile
        115

<210>  33
<211>  381
<212>  DNA
<213>  Enterobacter sakazakii

<220>
<221>  CDS
<222>  (1)..(381)

<400>  33
atg att cgc acc atg ttg cag ggc aaa ctg cac cgc gtc aaa gtt act      48
Met Ile Arg Thr Met Leu Gln Gly Lys Leu His Arg Val Lys Val Thr
1               5                   10                  15
cag gcg gac ctg cat tat gaa ggc tcc tgc gcc atc gat cag gat ttc      96
Gln Ala Asp Leu His Tyr Glu Gly Ser Cys Ala Ile Asp Gln Asp Phe
            20                  25                  30
ctc gag gcc gcg ggc att ctg gaa tat gaa gcc att gat atc tac aac      144
Leu Glu Ala Ala Gly Ile Leu Glu Tyr Glu Ala Ile Asp Ile Tyr Asn
        35                  40                  45
gtg acc aat ggc aaa cgc ttt tcg acg tac gcc atc gcg ggc gag cgc      192
Val Thr Asn Gly Lys Arg Phe Ser Thr Tyr Ala Ile Ala Gly Glu Arg
    50                  55                  60
ggc tcg aag atc att tcg gtg aac ggt gcc gcg gcc cac tgc gcg gat      240
Gly Ser Lys Ile Ile Ser Val Asn Gly Ala Ala Ala His Cys Ala Asp
65                  70                  75                  80
gtc ggc gat atc ctg att att gca agc tac gtg acc atg ccg gat gag      288
Val Gly Asp Ile Leu Ile Ile Ala Ser Tyr Val Thr Met Pro Asp Glu
                85                  90                  95
cag gcg cgc agc tgg cag ccg aaa gtt gcc tat ttc gac ggc gac aac      336
Gln Ala Arg Ser Trp Gln Pro Lys Val Ala Tyr Phe Asp Gly Asp Asn
            100                 105                 110
gag atg aaa cgt ctc gcc aaa gcg gtg ccg gtg cag gtg gcg taa          381
Glu Met Lys Arg Leu Ala Lys Ala Val Pro Val Gln Val Ala
        115                 120                 125

<210>  34
<211>  126
<212>  PRT
<213>  Enterobacter sakazakii

<400>  34
Met Ile Arg Thr Met Leu Gln Gly Lys Leu His Arg Val Lys Val Thr
1               5                   10                  15
Gln Ala Asp Leu His Tyr Glu Gly Ser Cys Ala Ile Asp Gln Asp Phe
            20                  25                  30
Leu Glu Ala Ala Gly Ile Leu Glu Tyr Glu Ala Ile Asp Ile Tyr Asn
        35                  40                  45
Val Thr Asn Gly Lys Arg Phe Ser Thr Tyr Ala Ile Ala Gly Glu Arg
    50                  55                  60
Gly Ser Lys Ile Ile Ser Val Asn Gly Ala Ala Ala His Cys Ala Asp
65                  70                  75                  80

Val Gly Asp Ile Leu Ile Ile Ala Ser Tyr Val Thr Met Pro Asp Glu
                85                  90                  95
Gln Ala Arg Ser Trp Gln Pro Lys Val Ala Tyr Phe Asp Gly Asp Asn
            100                 105                 110
Glu Met Lys Arg Leu Ala Lys Ala Val Pro Val Gln Val Ala
        115                 120                 125

<210>  35
<211>  351
<212>  DNA
<213>  Flavobacterium johnsoniae

<220>
<221>  CDS
<222>  (1)..(351)

<400>  35
atg caa att caa gtt ata aaa tca aaa att cat cga gta aaa gtt act        48
Met Gln Ile Gln Val Ile Lys Ser Lys Ile His Arg Val Lys Val Thr
1               5                   10                  15
gga gct gat tta aat tat att ggc agt att act att gat gaa act tta        96
Gly Ala Asp Leu Asn Tyr Ile Gly Ser Ile Thr Ile Asp Glu Thr Leu
            20                  25                  30
ctg gaa gcc tca aac att att gaa ggc gaa aaa gta tct att gtt aac       144
Leu Glu Ala Ser Asn Ile Ile Glu Gly Glu Lys Val Ser Ile Val Asn
        35                  40                  45
att aat aat gga gaa cgt ttt gaa act tac gcc ata aaa gga gaa aaa       192
Ile Asn Asn Gly Glu Arg Phe Glu Thr Tyr Ala Ile Lys Gly Glu Lys
    50                  55                  60
aat tca ggc gaa att aca ctg aac ggc cca gct gcc aga aag gtt caa       240
Asn Ser Gly Glu Ile Thr Leu Asn Gly Pro Ala Ala Arg Lys Val Gln
65                  70                  75                  80
aaa gac gac att att att atc att tcg tat gca acc ctt gat ttt gaa       288
Lys Asp Asp Ile Ile Ile Ile Ile Ser Tyr Ala Thr Leu Asp Phe Glu
                85                  90                  95
gaa gct aaa acc ttc aaa cca tgg atc att ttc cca aat gaa aac gat       336
Glu Ala Lys Thr Phe Lys Pro Trp Ile Ile Phe Pro Asn Glu Asn Asp
            100                 105                 110
aat tcg tta acc tag                                                   351
Asn Ser Leu Thr
        115

<210>  36
<211>  116
<212>  PRT
<213>  Flavobacterium johnsoniae

<400>  36
Met Gln Ile Gln Val Ile Lys Ser Lys Ile His Arg Val Lys Val Thr
1               5                   10                  15
Gly Ala Asp Leu Asn Tyr Ile Gly Ser Ile Thr Ile Asp Glu Thr Leu
            20                  25                  30
Leu Glu Ala Ser Asn Ile Ile Glu Gly Glu Lys Val Ser Ile Val Asn
        35                  40                  45
Ile Asn Asn Gly Glu Arg Phe Glu Thr Tyr Ala Ile Lys Gly Glu Lys
    50                  55                  60
Asn Ser Gly Glu Ile Thr Leu Asn Gly Pro Ala Ala Arg Lys Val Gln
65                  70                  75                  80
Lys Asp Asp Ile Ile Ile Ile Ile Ser Tyr Ala Thr Leu Asp Phe Glu
                85                  90                  95
Glu Ala Lys Thr Phe Lys Pro Trp Ile Ile Phe Pro Asn Glu Asn Asp

                100                 105                 110
Asn Ser Leu Thr
        115



<210>  37
<211>  351
<212>  DNA
<213>  Flavobacterium psychrophilum

<220>
<221>  CDS
<222>  (1)..(351)

<400>  37
atg caa att gaa gtt gta aaa tct aaa ata cac cga gta aca gta aca        48
Met Gln Ile Glu Val Val Lys Ser Lys Ile His Arg Val Thr Val Thr
1               5                   10                  15
ggc gcc gat tta aac tat atc gga agc att acc ata gac gaa gtt tta        96
Gly Ala Asp Leu Asn Tyr Ile Gly Ser Ile Thr Ile Asp Glu Val Leu
            20                  25                  30
ctc gaa gcg tct aac att att gaa ggc gaa aaa gta aac att gta aat       144
Leu Glu Ala Ser Asn Ile Ile Glu Gly Glu Lys Val Asn Ile Val Asn
        35                  40                  45
atc aat aac gga gaa cgt ttt gaa act tac gcc ata aaa ggc gca aaa       192
Ile Asn Asn Gly Glu Arg Phe Glu Thr Tyr Ala Ile Lys Gly Ala Lys
    50                  55                  60
aat tca ggc gaa att acc cta aat ggc ccc gca gct cgc aag gtt cat       240
Asn Ser Gly Glu Ile Thr Leu Asn Gly Pro Ala Ala Arg Lys Val His
65                  70                  75                  80
aaa gga gat ata att atc att atg tct tac gct cgc atg gat ttt gaa       288
Lys Gly Asp Ile Ile Ile Ile Met Ser Tyr Ala Arg Met Asp Phe Glu
                85                  90                  95
aaa gca aaa acc ttc aag cca tgg ttg gtt ttt cct aat gaa aaa gat       336
Lys Ala Lys Thr Phe Lys Pro Trp Leu Val Phe Pro Asn Glu Lys Asp
            100                 105                 110
aat tca ttg aaa taa                                                   351
Asn Ser Leu Lys
        115



<210>  38
<211>  116
<212>  PRT
<213>  Flavobacterium psychrophilum

<400>  38
Met Gln Ile Glu Val Val Lys Ser Lys Ile His Arg Val Thr Val Thr
1               5                   10                  15
Gly Ala Asp Leu Asn Tyr Ile Gly Ser Ile Thr Ile Asp Glu Val Leu
            20                  25                  30
Leu Glu Ala Ser Asn Ile Ile Glu Gly Glu Lys Val Asn Ile Val Asn
        35                  40                  45
Ile Asn Asn Gly Glu Arg Phe Glu Thr Tyr Ala Ile Lys Gly Ala Lys
    50                  55                  60
Asn Ser Gly Glu Ile Thr Leu Asn Gly Pro Ala Ala Arg Lys Val His
65                  70                  75                  80
Lys Gly Asp Ile Ile Ile Ile Met Ser Tyr Ala Arg Met Asp Phe Glu
                85                  90                  95
Lys Ala Lys Thr Phe Lys Pro Trp Leu Val Phe Pro Asn Glu Lys Asp
            100                 105                 110
Asn Ser Leu Lys
        115

<210>  39
<211>  381
<212>  DNA
<213>  Klebsiella pneumoniae

<220>
<221>  CDS
<222>  (1)..(381)

<400>  39
atg atg cgt aat atg ctg cag ggc aag ctc cac cgc gta aaa gtc acg      48
Met Met Arg Asn Met Leu Gln Gly Lys Leu His Arg Val Lys Val Thr
1               5                   10                  15
cag gcg gac ctg cac tat gaa ggt tct tgc gcc att gac cag gac ttc      96
Gln Ala Asp Leu His Tyr Glu Gly Ser Cys Ala Ile Asp Gln Asp Phe
            20                  25                  30
ctg gat gcg gcc ggt att ctg gag aat gaa act att cac ctc tgg aac     144
Leu Asp Ala Ala Gly Ile Leu Glu Asn Glu Thr Ile His Leu Trp Asn
        35                  40                  45
gtc acc aac ggc aac cgt ttc tcc acc tac gcc att gcc gcc gag cgg     192
Val Thr Asn Gly Asn Arg Phe Ser Thr Tyr Ala Ile Ala Ala Glu Arg
    50                  55                  60
ggc tcc cgg atc atc tcc gtt aac ggc gcc gcc gcg cac tgc gcc agc     240
Gly Ser Arg Ile Ile Ser Val Asn Gly Ala Ala Ala His Cys Ala Ser
65                  70                  75                  80
gtc ggc gat att ttg atc atc gcc agc ttc gtt acc atg tcg gac gaa     288
Val Gly Asp Ile Leu Ile Ile Ala Ser Phe Val Thr Met Ser Asp Glu
                85                  90                  95
gag gcg cgc agc tgg cag cct aac atc gcc tac ttc gaa ggc gac aac     336
Glu Ala Arg Ser Trp Gln Pro Asn Ile Ala Tyr Phe Glu Gly Asp Asn
            100                 105                 110
gaa atg aag cgc cag gca aaa gct att ccg gta cag gtc gcc tga         381
Glu Met Lys Arg Gln Ala Lys Ala Ile Pro Val Gln Val Ala
        115                 120                 125


<210>  40
<211>  126
<212>  PRT
<213>  Klebsiella pneumoniae

<400>  40
Met Met Arg Asn Met Leu Gln Gly Lys Leu His Arg Val Lys Val Thr
1               5                   10                  15
Gln Ala Asp Leu His Tyr Glu Gly Ser Cys Ala Ile Asp Gln Asp Phe
            20                  25                  30
Leu Asp Ala Ala Gly Ile Leu Glu Asn Glu Thr Ile His Leu Trp Asn
        35                  40                  45
Val Thr Asn Gly Asn Arg Phe Ser Thr Tyr Ala Ile Ala Ala Glu Arg
    50                  55                  60
Gly Ser Arg Ile Ile Ser Val Asn Gly Ala Ala Ala His Cys Ala Ser
65                  70                  75                  80
Val Gly Asp Ile Leu Ile Ile Ala Ser Phe Val Thr Met Ser Asp Glu
                85                  90                  95
Glu Ala Arg Ser Trp Gln Pro Asn Ile Ala Tyr Phe Glu Gly Asp Asn
            100                 105                 110
Glu Met Lys Arg Gln Ala Lys Ala Ile Pro Val Gln Val Ala
        115                 120                 125


<210>  41

<211>   381
<212>   DNA
<213>   Methylophilus methylotrophus

<220>
<221>   CDS
<222>   (1)..(381)

<400>   41
atg caa aga acc atg ttg aaa tca aag ctg cat cgg gtg cgt gtg acg        48
Met Gln Arg Thr Met Leu Lys Ser Lys Leu His Arg Val Arg Val Thr
1               5                   10                  15
cat agc gag ctg cac tat gaa ggc agc tgt gcg att gat gag gcc ttg        96
His Ser Glu Leu His Tyr Glu Gly Ser Cys Ala Ile Asp Glu Ala Leu
            20                  25                  30
ctg gaa gct gcc aat atc cac gaa tat gag caa atc cag att tac aac       144
Leu Glu Ala Ala Asn Ile His Glu Tyr Glu Gln Ile Gln Ile Tyr Asn
        35                  40                  45
atc aac aat ggt gag cgt ttt acg act tac gcc atc cgt gcc gag cgt       192
Ile Asn Asn Gly Glu Arg Phe Thr Thr Tyr Ala Ile Arg Ala Glu Arg
    50                  55                  60
cat tct ggt gtg att tca gtc aat ggt gcg gct gca cat aaa gcc gat       240
His Ser Gly Val Ile Ser Val Asn Gly Ala Ala Ala His Lys Ala Asp
65                  70                  75                  80
ccg gat gat ttg att att att gcc agt tat tcg caa tat acc gaa gcc       288
Pro Asp Asp Leu Ile Ile Ile Ala Ser Tyr Ser Gln Tyr Thr Glu Ala
                85                  90                  95
gaa ctg tcc aat tac cat ccg caa ctg gtc tac gtg gat gcg caa aac       336
Glu Leu Ser Asn Tyr His Pro Gln Leu Val Tyr Val Asp Ala Gln Asn
            100                 105                 110
cgc att gtt gaa cag aaa aac cat att ccc gcg caa gcc gcc tga           381
Arg Ile Val Glu Gln Lys Asn His Ile Pro Ala Gln Ala Ala
        115                 120                 125


<210>   42
<211>   126
<212>   PRT
<213>   Methylophilus methylotrophus

<400>   42
Met Gln Arg Thr Met Leu Lys Ser Lys Leu His Arg Val Arg Val Thr
1               5                   10                  15
His Ser Glu Leu His Tyr Glu Gly Ser Cys Ala Ile Asp Glu Ala Leu
            20                  25                  30
Leu Glu Ala Ala Asn Ile His Glu Tyr Glu Gln Ile Gln Ile Tyr Asn
        35                  40                  45
Ile Asn Asn Gly Glu Arg Phe Thr Thr Tyr Ala Ile Arg Ala Glu Arg
    50                  55                  60
His Ser Gly Val Ile Ser Val Asn Gly Ala Ala Ala His Lys Ala Asp
65                  70                  75                  80
Pro Asp Asp Leu Ile Ile Ile Ala Ser Tyr Ser Gln Tyr Thr Glu Ala
                85                  90                  95
Glu Leu Ser Asn Tyr His Pro Gln Leu Val Tyr Val Asp Ala Gln Asn
            100                 105                 110
Arg Ile Val Glu Gln Lys Asn His Ile Pro Ala Gln Ala Ala
        115                 120                 125


<210>   43
<211>   432
<212>   DNA
<213>   Mycobacterium avium

<220>
<221>  CDS
<222>  (1)..(432)

<400>  43
atg tta cgg acg atg ctc aag tcg aag atc cac cgg gcc acc gtc acg        48
Met Leu Arg Thr Met Leu Lys Ser Lys Ile His Arg Ala Thr Val Thr
1               5                   10                  15
cag gcc gac ctg cac tac gtg ggc tcg gtg acc atc gac gcc gac ctg        96
Gln Ala Asp Leu His Tyr Val Gly Ser Val Thr Ile Asp Ala Asp Leu
            20                  25                  30
atg gac gcc gcc gac ctg ctc gag ggc gaa cag gtg acc atc gtc gac       144
Met Asp Ala Ala Asp Leu Leu Glu Gly Glu Gln Val Thr Ile Val Asp
        35                  40                  45
atc gac aac ggc gcc cgg ctg gtc acc tac gcg atc acc ggt gag cgt       192
Ile Asp Asn Gly Ala Arg Leu Val Thr Tyr Ala Ile Thr Gly Glu Arg
    50                  55                  60
ggc agc ggg gtg atc ggg atc aac ggt gcc gca gcg cat ctg gtc cac       240
Gly Ser Gly Val Ile Gly Ile Asn Gly Ala Ala Ala His Leu Val His
65                  70                  75                  80
ccg ggc gat ctg gtc atc ctg atc gcg tat ggg acc atg gag gag gcc       288
Pro Gly Asp Leu Val Ile Leu Ile Ala Tyr Gly Thr Met Glu Glu Ala
                85                  90                  95
gag gcg cgg gcg tat cag ccg cga atc gtc ttc gtc gac gcc gac aac       336
Glu Ala Arg Ala Tyr Gln Pro Arg Ile Val Phe Val Asp Ala Asp Asn
            100                 105                 110
aag ccg gtc gac ctc ggc cac gat ccg gca ttc gtg ccg gac ttc gag       384
Lys Pro Val Asp Leu Gly His Asp Pro Ala Phe Val Pro Asp Phe Glu
        115                 120                 125
ata gca ggg gcg gcc gag ctg ctc gat ccc cgg atc gtt gcg cgg tag       432
Ile Ala Gly Ala Ala Glu Leu Leu Asp Pro Arg Ile Val Ala Arg
    130                 135                 140


<210>  44
<211>  143
<212>  PRT
<213>  Mycobacterium avium

<400>  44
Met Leu Arg Thr Met Leu Lys Ser Lys Ile His Arg Ala Thr Val Thr
1               5                   10                  15
Gln Ala Asp Leu His Tyr Val Gly Ser Val Thr Ile Asp Ala Asp Leu
            20                  25                  30
Met Asp Ala Ala Asp Leu Leu Glu Gly Glu Gln Val Thr Ile Val Asp
        35                  40                  45
Ile Asp Asn Gly Ala Arg Leu Val Thr Tyr Ala Ile Thr Gly Glu Arg
    50                  55                  60
Gly Ser Gly Val Ile Gly Ile Asn Gly Ala Ala Ala His Leu Val His
65                  70                  75                  80
Pro Gly Asp Leu Val Ile Leu Ile Ala Tyr Gly Thr Met Glu Glu Ala
                85                  90                  95
Glu Ala Arg Ala Tyr Gln Pro Arg Ile Val Phe Val Asp Ala Asp Asn
            100                 105                 110
Lys Pro Val Asp Leu Gly His Asp Pro Ala Phe Val Pro Asp Phe Glu
        115                 120                 125
Ile Ala Gly Ala Ala Glu Leu Leu Asp Pro Arg Ile Val Ala Arg
    130                 135                 140


<210>  45
<211>  351

<212>  DNA
<213>  Parabacteroides distasonis

<220>
<221>  CDS
<222>  (1)..(351)

<400>  45
atg ttt ata gag gta gta aag tca aaa att cac cgg gta acg gta acg      48
Met Phe Ile Glu Val Val Lys Ser Lys Ile His Arg Val Thr Val Thr
1               5                   10                  15
gaa gct aac tta aac tat atc ggt agc atc act atc gac gag gat ttg      96
Glu Ala Asn Leu Asn Tyr Ile Gly Ser Ile Thr Ile Asp Glu Asp Leu
            20                  25                  30
ttg gac gcc gct aat ttg atc gcg aac gag aag gtc tcg atc gtg aat     144
Leu Asp Ala Ala Asn Leu Ile Ala Asn Glu Lys Val Ser Ile Val Asn
        35                  40                  45
aat aat aac gga gaa cgt ttt gag acg tat att atc aag ggc gaa cgt     192
Asn Asn Asn Gly Glu Arg Phe Glu Thr Tyr Ile Ile Lys Gly Glu Arg
    50                  55                  60
ggc tcg gga gtg gtt tgc ttg aac gga gcg gct gcc cgt aag gct caa     240
Gly Ser Gly Val Val Cys Leu Asn Gly Ala Ala Ala Arg Lys Ala Gln
65                  70                  75                  80
ccc gga gat att att atc gtt atg tcg tat gcc atg atg gat ttc gag     288
Pro Gly Asp Ile Ile Ile Val Met Ser Tyr Ala Met Met Asp Phe Glu
                85                  90                  95
gag gcg aaa aca ttc aag cct tcc gtg gta ttt ccc gat acg gcg aca     336
Glu Ala Lys Thr Phe Lys Pro Ser Val Val Phe Pro Asp Thr Ala Thr
            100                 105                 110
aat aag ttg ata tag                                                 351
Asn Lys Leu Ile
        115


<210>  46
<211>  116
<212>  PRT
<213>  Parabacteroides distasonis

<400>  46
Met Phe Ile Glu Val Val Lys Ser Lys Ile His Arg Val Thr Val Thr
1               5                   10                  15
Glu Ala Asn Leu Asn Tyr Ile Gly Ser Ile Thr Ile Asp Glu Asp Leu
            20                  25                  30
Leu Asp Ala Ala Asn Leu Ile Ala Asn Glu Lys Val Ser Ile Val Asn
        35                  40                  45
Asn Asn Asn Gly Glu Arg Phe Glu Thr Tyr Ile Ile Lys Gly Glu Arg
    50                  55                  60
Gly Ser Gly Val Val Cys Leu Asn Gly Ala Ala Ala Arg Lys Ala Gln
65                  70                  75                  80
Pro Gly Asp Ile Ile Ile Val Met Ser Tyr Ala Met Met Asp Phe Glu
                85                  90                  95
Glu Ala Lys Thr Phe Lys Pro Ser Val Val Phe Pro Asp Thr Ala Thr
            100                 105                 110
Asn Lys Leu Ile
        115


<210>  47
<211>  357
<212>  DNA
<213>  Porphyromonas gingivalis

<220>
<221> CDS
<222> (1)..(357)

<400> 47
atg tac gtc gaa gta tta aaa tcc aaa ata cac cgc gta acc att acg 48
Met Tyr Val Glu Val Leu Lys Ser Lys Ile His Arg Val Thr Ile Thr
1               5                   10                  15
gag gcc aac ctc aac tat gtg ggt agc atc acc atc gat gaa gat ctg 96
Glu Ala Asn Leu Asn Tyr Val Gly Ser Ile Thr Ile Asp Glu Asp Leu
            20                  25                  30
atg gat gct gcc aac atc atc gcc gga gag aaa gtc caa atc gtg gac 144
Met Asp Ala Ala Asn Ile Ile Ala Gly Glu Lys Val Gln Ile Val Asp
        35                  40                  45
aac aac aac gga gca cgc ttg gag acc tat acc ata ccg ggc aaa aga 192
Asn Asn Asn Gly Ala Arg Leu Glu Thr Tyr Thr Ile Pro Gly Lys Arg
    50                  55                  60
ggt tcg ggg gta atc tgt ctc aat gga gca gct gcc cgc atc gtt cat 240
Gly Ser Gly Val Ile Cys Leu Asn Gly Ala Ala Ala Arg Ile Val His
65                  70                  75                  80
ccg ggc gac atc atc atc atc atg gca tat gcc tgg atg ccg atg gag 288
Pro Gly Asp Ile Ile Ile Ile Met Ala Tyr Ala Trp Met Pro Met Glu
                85                  90                  95
gaa gcc aag gtg ttc aag cct gct gta gta ttt ccc gat act gca acg 336
Glu Ala Lys Val Phe Lys Pro Ala Val Val Phe Pro Asp Thr Ala Thr
            100                 105                 110
aac aag ata atc ctg aag taa 357
Asn Lys Ile Ile Leu Lys
        115

<210> 48
<211> 118
<212> PRT
<213> Porphyromonas gingivalis

<400> 48
Met Tyr Val Glu Val Leu Lys Ser Lys Ile His Arg Val Thr Ile Thr
1               5                   10                  15
Glu Ala Asn Leu Asn Tyr Val Gly Ser Ile Thr Ile Asp Glu Asp Leu
            20                  25                  30
Met Asp Ala Ala Asn Ile Ile Ala Gly Glu Lys Val Gln Ile Val Asp
        35                  40                  45
Asn Asn Asn Gly Ala Arg Leu Glu Thr Tyr Thr Ile Pro Gly Lys Arg
    50                  55                  60
Gly Ser Gly Val Ile Cys Leu Asn Gly Ala Ala Ala Arg Ile Val His
65                  70                  75                  80
Pro Gly Asp Ile Ile Ile Ile Met Ala Tyr Ala Trp Met Pro Met Glu
                85                  90                  95
Glu Ala Lys Val Phe Lys Pro Ala Val Val Phe Pro Asp Thr Ala Thr
            100                 105                 110
Asn Lys Ile Ile Leu Lys
        115

<210> 49
<211> 447
<212> DNA
<213> Rhodococcus sp.

<220>
<221> CDS
<222> (1)..(447)

<400>  49
atg ttt cgc acc atg atg aag tcg aag atc cat cgc gcc acc gtc acg      48
Met Phe Arg Thr Met Met Lys Ser Lys Ile His Arg Ala Thr Val Thr
1               5                   10                  15
cac gcc gac ctg cac tac gtg ggc tcg gtg acg gtc gac cag gat ctg      96
His Ala Asp Leu His Tyr Val Gly Ser Val Thr Val Asp Gln Asp Leu
            20                  25                  30
atg gac gcc gcc gac ctt ctc gag ggc gag cag gtg tgc atc gtc gac      144
Met Asp Ala Ala Asp Leu Leu Glu Gly Glu Gln Val Cys Ile Val Asp
        35                  40                  45
atc gac aac ggc gcg cgg ctc gaa acg tat gtg atc gcc ggc gag cgg      192
Ile Asp Asn Gly Ala Arg Leu Glu Thr Tyr Val Ile Ala Gly Glu Arg
    50                  55                  60
gga agt ggt gtc atc ggg atc aac ggt gcg gca gcg cat ctg gtc aag      240
Gly Ser Gly Val Ile Gly Ile Asn Gly Ala Ala Ala His Leu Val Lys
65                  70                  75                  80
ccc ggc gac ctc gtc atc ctc atc gcg tac ggc gtc atg aac gag cag      288
Pro Gly Asp Leu Val Ile Leu Ile Ala Tyr Gly Val Met Asn Glu Gln
                85                  90                  95
gag gtg cgc gac tac gag ccg cgt gtc gtg ttc gtc gac gcc gcc aac      336
Glu Val Arg Asp Tyr Glu Pro Arg Val Val Phe Val Asp Ala Ala Asn
            100                 105                 110
tca ccg atc gag ctc ggc gcc gac ccc gcg cac gcg ccg gag ggc tcc      384
Ser Pro Ile Glu Leu Gly Ala Asp Pro Ala His Ala Pro Glu Gly Ser
        115                 120                 125
ggt ctc atc acc ccg cgc atg ctg tcg acg ctc gac gcc gag cgc gaa      432
Gly Leu Ile Thr Pro Arg Met Leu Ser Thr Leu Asp Ala Glu Arg Glu
    130                 135                 140
tcc agt ttg gtg taa                                                  447
Ser Ser Leu Val
145


<210>  50
<211>  148
<212>  PRT
<213>  Rhodococcus sp.

<400>  50
Met Phe Arg Thr Met Met Lys Ser Lys Ile His Arg Ala Thr Val Thr
1               5                   10                  15
His Ala Asp Leu His Tyr Val Gly Ser Val Thr Val Asp Gln Asp Leu
            20                  25                  30
Met Asp Ala Ala Asp Leu Leu Glu Gly Glu Gln Val Cys Ile Val Asp
        35                  40                  45
Ile Asp Asn Gly Ala Arg Leu Glu Thr Tyr Val Ile Ala Gly Glu Arg
    50                  55                  60
Gly Ser Gly Val Ile Gly Ile Asn Gly Ala Ala Ala His Leu Val Lys
65                  70                  75                  80
Pro Gly Asp Leu Val Ile Leu Ile Ala Tyr Gly Val Met Asn Glu Gln
                85                  90                  95
Glu Val Arg Asp Tyr Glu Pro Arg Val Val Phe Val Asp Ala Ala Asn
            100                 105                 110
Ser Pro Ile Glu Leu Gly Ala Asp Pro Ala His Ala Pro Glu Gly Ser
        115                 120                 125
Gly Leu Ile Thr Pro Arg Met Leu Ser Thr Leu Asp Ala Glu Arg Glu
    130                 135                 140
Ser Ser Leu Val
145


<210>  51

<211>  492
<212>  DNA
<213>  Saccharopolyspora erythraea

<220>
<221>  CDS
<222>  (1)..(492)

<400>  51
atg ttc cgc acg atg ctg aag tcc aag atc cac cga gcc acg gtc acc        48
Met Phe Arg Thr Met Leu Lys Ser Lys Ile His Arg Ala Thr Val Thr
1               5                   10                  15
cag gcc gac ctg cac tac gtc ggg tcg gtc acc gtt gac gcc gac ctg        96
Gln Ala Asp Leu His Tyr Val Gly Ser Val Thr Val Asp Ala Asp Leu
            20                  25                  30
atg gac gcc gcc gac ctg ctc gaa ggc gag cag gtg gcg atc gtc gac       144
Met Asp Ala Ala Asp Leu Leu Glu Gly Glu Gln Val Ala Ile Val Asp
        35                  40                  45
gtc acc aac ggc gcg cgg ctg gag acc tac gtg atc acc ggc gag cgc       192
Val Thr Asn Gly Ala Arg Leu Glu Thr Tyr Val Ile Thr Gly Glu Arg
    50                  55                  60
ggc tcg ggc gtg atc ggc atc aac ggc gcc gcc gcg cac ctg atc gag       240
Gly Ser Gly Val Ile Gly Ile Asn Gly Ala Ala Ala His Leu Ile Glu
65                  70                  75                  80
ccc ggc gac ctg gtg atc ctg atc tcc tac ggg gtg atg gac gag ctg       288
Pro Gly Asp Leu Val Ile Leu Ile Ser Tyr Gly Val Met Asp Glu Leu
                85                  90                  95
gag gcg cgg tcg gtg cgg ccg aag gtg atc ttc gtc gac gcc gac aac       336
Glu Ala Arg Ser Val Arg Pro Lys Val Ile Phe Val Asp Ala Asp Asn
            100                 105                 110
agg atc gtc gag cgc ggc cag gac ccg ggc cac gcc ccg gcg ggc tcc       384
Arg Ile Val Glu Arg Gly Gln Asp Pro Gly His Ala Pro Ala Gly Ser
        115                 120                 125
ggg ctg gcc ggc acg gcc gcc tcg gtc acc tcg gcg atc acc gag gcc       432
Gly Leu Ala Gly Thr Ala Ala Ser Val Thr Ser Ala Ile Thr Glu Ala
    130                 135                 140
gcc gcc gag acc gac gac gcg gcc aag ctc gac gcc ctg ctc cag cag       480
Ala Ala Glu Thr Asp Asp Ala Ala Lys Leu Asp Ala Leu Leu Gln Gln
145                 150                 155                 160
ccg gag cac tga                                                       492
Pro Glu His

<210>  52
<211>  163
<212>  PRT
<213>  Saccharopolyspora erythraea

<400>  52
Met Phe Arg Thr Met Leu Lys Ser Lys Ile His Arg Ala Thr Val Thr
1               5                   10                  15
Gln Ala Asp Leu His Tyr Val Gly Ser Val Thr Val Asp Ala Asp Leu
            20                  25                  30
Met Asp Ala Ala Asp Leu Leu Glu Gly Glu Gln Val Ala Ile Val Asp
        35                  40                  45
Val Thr Asn Gly Ala Arg Leu Glu Thr Tyr Val Ile Thr Gly Glu Arg
    50                  55                  60
Gly Ser Gly Val Ile Gly Ile Asn Gly Ala Ala Ala His Leu Ile Glu
65                  70                  75                  80
Pro Gly Asp Leu Val Ile Leu Ile Ser Tyr Gly Val Met Asp Glu Leu
                85                  90                  95
Glu Ala Arg Ser Val Arg Pro Lys Val Ile Phe Val Asp Ala Asp Asn

            100                 105                 110
Arg Ile Val Glu Arg Gly Gln Asp Pro Gly His Ala Pro Ala Gly Ser
        115                 120                 125
Gly Leu Ala Gly Thr Ala Ala Ser Val Thr Ser Ala Ile Thr Glu Ala
    130                 135                 140
Ala Ala Glu Thr Asp Asp Ala Ala Lys Leu Asp Ala Leu Leu Gln Gln
145                 150                 155                 160
Pro Glu His

<210> 53
<211> 441
<212> DNA
<213> Salinibacter ruber

<220>
<221> CDS
<222> (1)..(441)

<400> 53
atg gac ctt tcg atg ctc agg gca aag ctc cac cgg ctt cgc gtc acc 48
Met Asp Leu Ser Met Leu Arg Ala Lys Leu His Arg Leu Arg Val Thr
1               5                   10                  15
gag gcc gac ctg tat tat gag ggg tcg att acc atc gac gag gag ctg 96
Glu Ala Asp Leu Tyr Tyr Glu Gly Ser Ile Thr Ile Asp Glu Glu Leu
            20                  25                  30
ttg gac gcc gcc ggt ctg ctg ccc tac gag aag gtg cag gtg gtg aac 144
Leu Asp Ala Ala Gly Leu Leu Pro Tyr Glu Lys Val Gln Val Val Asn
        35                  40                  45
gtg aac aat ggc agt cgg ctc gaa acc tac acg att ccg ggg gag gcg 192
Val Asn Asn Gly Ser Arg Leu Glu Thr Tyr Thr Ile Pro Gly Glu Ala
    50                  55                  60
ggg gag cgc aca gtg tgt cta aac ggc ccg gcg gcc cgg ctg gcg gcc 240
Gly Glu Arg Thr Val Cys Leu Asn Gly Pro Ala Ala Arg Leu Ala Ala
65                  70                  75                  80
ccg gga gac gaa gtc atc gtg atc gcg tac gcc gaa ctt acc cct tct 288
Pro Gly Asp Glu Val Ile Val Ile Ala Tyr Ala Glu Leu Thr Pro Ser
                85                  90                  95
gag gcc cgc gag cat cac cca cgc gtg gtg cac gtc gac gag aac aac 336
Glu Ala Arg Glu His His Pro Arg Val Val His Val Asp Glu Asn Asn
            100                 105                 110
gac gtg acg aag aca cga acc ctc gac gtc gcg aag gaa acg gac gaa 384
Asp Val Thr Lys Thr Arg Thr Leu Asp Val Ala Lys Glu Thr Asp Glu
        115                 120                 125
aac ctc gcg ccg gac ggc atg gaa gac gtc ctc att gcc gag ggg ccg 432
Asn Leu Ala Pro Asp Gly Met Glu Asp Val Leu Ile Ala Glu Gly Pro
    130                 135                 140
cag tca tga 441
Gln Ser
145

<210> 54
<211> 146
<212> PRT
<213> Salinibacter ruber

<400> 54
Met Asp Leu Ser Met Leu Arg Ala Lys Leu His Arg Leu Arg Val Thr
1               5                   10                  15
Glu Ala Asp Leu Tyr Tyr Glu Gly Ser Ile Thr Ile Asp Glu Glu Leu
            20                  25                  30

Leu Asp Ala Ala Gly Leu Leu Pro Tyr Glu Lys Val Gln Val Val Asn
        35                  40                  45
Val Asn Asn Gly Ser Arg Leu Glu Thr Tyr Thr Ile Pro Gly Glu Ala
    50                  55                  60
Gly Glu Arg Thr Val Cys Leu Asn Gly Pro Ala Ala Arg Leu Ala Ala
65                  70                  75                  80
Pro Gly Asp Glu Val Ile Val Ile Ala Tyr Ala Glu Leu Thr Pro Ser
                85                  90                  95
Glu Ala Arg Glu His His Pro Arg Val Val His Val Asp Glu Asn Asn
            100                 105                 110
Asp Val Thr Lys Thr Arg Thr Leu Asp Val Ala Lys Glu Thr Asp Glu
        115                 120                 125
Asn Leu Ala Pro Asp Gly Met Glu Asp Val Leu Ile Ala Glu Gly Pro
    130                 135                 140
Gln Ser
145

<210>  55
<211>  381
<212>  DNA
<213>  Salmonella enterica

<220>
<221>  CDS
<222>  (1)..(381)

<400>  55
atg att cgc aca atg tta caa ggt aag ctc cat cgc gtc aaa gtg acc      48
Met Ile Arg Thr Met Leu Gln Gly Lys Leu His Arg Val Lys Val Thr
1               5                   10                  15
cag gct gac ctg cat tac gag ggt tcc tgc gct att gac cag gat ttt      96
Gln Ala Asp Leu His Tyr Glu Gly Ser Cys Ala Ile Asp Gln Asp Phe
            20                  25                  30
ctc gac gcg gcc gga att ctg gag aac gaa gcc att gat atc tgg aat      144
Leu Asp Ala Ala Gly Ile Leu Glu Asn Glu Ala Ile Asp Ile Trp Asn
        35                  40                  45
gtg tcc aat ggt aag cgc ttc tca acc tat gcg att gcg gcg gag cgt      192
Val Ser Asn Gly Lys Arg Phe Ser Thr Tyr Ala Ile Ala Ala Glu Arg
    50                  55                  60
ggc tct aaa att atc tcc gtt aac ggg gca gcg gca cat tgc gcc tcc      240
Gly Ser Lys Ile Ile Ser Val Asn Gly Ala Ala Ala His Cys Ala Ser
65                  70                  75                  80
gtg ggt gat att gtc att att gcc agc ttt gtc acc atg agc gat gaa      288
Val Gly Asp Ile Val Ile Ile Ala Ser Phe Val Thr Met Ser Asp Glu
                85                  90                  95
gaa gcg cgc acc tgg cac ccg aac gtt gcc tac ttc gaa ggc gac aac      336
Glu Ala Arg Thr Trp His Pro Asn Val Ala Tyr Phe Glu Gly Asp Asn
            100                 105                 110
gaa atg aaa cgc acc gca aaa gcc att ccg gta cag gtt gcc tga          381
Glu Met Lys Arg Thr Ala Lys Ala Ile Pro Val Gln Val Ala
        115                 120                 125

<210>  56
<211>  126
<212>  PRT
<213>  Salmonella enterica

<400>  56
Met Ile Arg Thr Met Leu Gln Gly Lys Leu His Arg Val Lys Val Thr
1               5                   10                  15
Gln Ala Asp Leu His Tyr Glu Gly Ser Cys Ala Ile Asp Gln Asp Phe

                    20                      25                      30
Leu Asp Ala Ala Gly Ile Leu Glu Asn Glu Ala Ile Asp Ile Trp Asn
        35                      40                      45
Val Ser Asn Gly Lys Arg Phe Ser Thr Tyr Ala Ile Ala Ala Glu Arg
    50                      55                      60
Gly Ser Lys Ile Ile Ser Val Asn Gly Ala Ala Ala His Cys Ala Ser
65                      70                      75                      80
Val Gly Asp Ile Val Ile Ile Ala Ser Phe Val Thr Met Ser Asp Glu
                85                      90                      95
Glu Ala Arg Thr Trp His Pro Asn Val Ala Tyr Phe Glu Gly Asp Asn
            100                     105                     110
Glu Met Lys Arg Thr Ala Lys Ala Ile Pro Val Gln Val Ala
        115                     120                     125

<210>  57
<211>  420
<212>  DNA
<213>  Streptomyces coelicolor

<220>
<221>  CDS
<222>  (1)..(420)

<400>  57
gtg ctg cga act ttg atc aag tcc aag atc cac cgc gcc acc gtc acc      48
Met Leu Arg Thr Leu Ile Lys Ser Lys Ile His Arg Ala Thr Val Thr
1               5                   10                  15
cag gcc gac ctg cac tac gtc ggc tcg gtg acc atc gac gcc gat ctc      96
Gln Ala Asp Leu His Tyr Val Gly Ser Val Thr Ile Asp Ala Asp Leu
            20                  25                  30
ctg gac gct gcc gat ctg ctg ccc ggt gag ctc gtg cac atc gtg gac      144
Leu Asp Ala Ala Asp Leu Leu Pro Gly Glu Leu Val His Ile Val Asp
        35                  40                  45
gtc acc aac ggg gcc cgg ctg gag acg tac gtc atc gag ggg gag cgc      192
Val Thr Asn Gly Ala Arg Leu Glu Thr Tyr Val Ile Glu Gly Glu Arg
    50                  55                  60
ggg tcc ggg gtg atc ggg atc aac ggg gcc gcg gcc cac ctc gtg cac      240
Gly Ser Gly Val Ile Gly Ile Asn Gly Ala Ala Ala His Leu Val His
65                  70                  75                  80
ccc ggt gac ctg gtg atc ctc atc agc tac gcc cag gtc acc gac gcc      288
Pro Gly Asp Leu Val Ile Leu Ile Ser Tyr Ala Gln Val Thr Asp Ala
                85                  90                  95
gag gcg cgg tcg ctg cgg ccc cgg gtc gtg cac gtg gac ggg gac aac      336
Glu Ala Arg Ser Leu Arg Pro Arg Val Val His Val Asp Gly Asp Asn
            100                 105                 110
cgt atc gtc ggc ctc ggc gcc gac gcg tcg gag ccg gta ccg ggc tcg      384
Arg Ile Val Gly Leu Gly Ala Asp Ala Ser Glu Pro Val Pro Gly Ser
        115                 120                 125
gac cag gag cgc agc cct cag gcc gtg tcg gcc tga                      420
Asp Gln Glu Arg Ser Pro Gln Ala Val Ser Ala
    130                 135

<210>  58
<211>  139
<212>  PRT
<213>  Streptomyces coelicolor

<400>  58
Met Leu Arg Thr Leu Ile Lys Ser Lys Ile His Arg Ala Thr Val Thr
1               5                   10                  15
Gln Ala Asp Leu His Tyr Val Gly Ser Val Thr Ile Asp Ala Asp Leu

20                  25                  30
Leu Asp Ala Ala Asp Leu Leu Pro Gly Glu Leu Val His Ile Val Asp
        35                  40                  45
Val Thr Asn Gly Ala Arg Leu Glu Thr Tyr Val Ile Glu Gly Glu Arg
    50                  55                  60
Gly Ser Gly Val Ile Gly Ile Asn Gly Ala Ala Ala His Leu Val His
65                  70                  75                  80
Pro Gly Asp Leu Val Ile Leu Ile Ser Tyr Ala Gln Val Thr Asp Ala
                85                  90                  95
Glu Ala Arg Ser Leu Arg Pro Arg Val Val His Val Asp Gly Asp Asn
            100                 105                 110
Arg Ile Val Gly Leu Gly Ala Asp Ala Ser Glu Pro Val Pro Gly Ser
        115                 120                 125
Asp Gln Glu Arg Ser Pro Gln Ala Val Ser Ala
    130                 135

<210>  59
<211>  414
<212>  DNA
<213>  Thermobifida fusca

<220>
<221>  CDS
<222>  (1)..(414)

<400>  59
gtg ctg cgc acc atg ctc aac gga aag atc cac cgg gcc acg gtc acc        48
Met Leu Arg Thr Met Leu Asn Gly Lys Ile His Arg Ala Thr Val Thr
1               5                   10                  15
cac gcg gac ctg cac tac gtc ggt tcc atc acc atc gac gcc gat ctg        96
His Ala Asp Leu His Tyr Val Gly Ser Ile Thr Ile Asp Ala Asp Leu
            20                  25                  30
atg gac gcg gcc aac ctc gtc gac ggc gaa cag gtc cac gtg gtc aac       144
Met Asp Ala Ala Asn Leu Val Asp Gly Glu Gln Val His Val Val Asn
        35                  40                  45
atc acg aac ggg cac cgg ttg gtc acc tac gtg ctg acc ggg gag cgc       192
Ile Thr Asn Gly His Arg Leu Val Thr Tyr Val Leu Thr Gly Glu Arg
    50                  55                  60
ggc agc ggt gtc atc gga atc aac ggg gcc gcc gcc cgc ctc gta gcg       240
Gly Ser Gly Val Ile Gly Ile Asn Gly Ala Ala Ala Arg Leu Val Ala
65                  70                  75                  80
ccg gga gac ctg gtg atc atc att tcg tat gtg cag ctc acc gag gct       288
Pro Gly Asp Leu Val Ile Ile Ile Ser Tyr Val Gln Leu Thr Glu Ala
                85                  90                  95
gag cgt gcc acc cac cgg ccc cac gtg gtg cac gtg gac gcc aac aac       336
Glu Arg Ala Thr His Arg Pro His Val Val His Val Asp Ala Asn Asn
            100                 105                 110
cgc atc gtc gcc ctg ggc gac gac ctg gct gag ccg gtc ccc gga agc       384
Arg Ile Val Ala Leu Gly Asp Asp Leu Ala Glu Pro Val Pro Gly Ser
        115                 120                 125
gac cag aaa tcg ggg gcg ctt ctc ccc taa                               414
Asp Gln Lys Ser Gly Ala Leu Leu Pro
    130                 135

<210>  60
<211>  137
<212>  PRT
<213>  Thermobifida fusca

<400>  60
Met Leu Arg Thr Met Leu Asn Gly Lys Ile His Arg Ala Thr Val Thr

1                   5                   10                  15
His Ala Asp Leu His Tyr Val Gly Ser Ile Thr Ile Asp Ala Asp Leu
                20                  25                  30
Met Asp Ala Ala Asn Leu Val Asp Gly Glu Gln Val His Val Val Asn
        35                  40                  45
Ile Thr Asn Gly His Arg Leu Val Thr Tyr Val Leu Thr Gly Glu Arg
    50                  55                  60
Gly Ser Gly Val Ile Gly Ile Asn Gly Ala Ala Ala Arg Leu Val Ala
65                  70                  75                  80
Pro Gly Asp Leu Val Ile Ile Ile Ser Tyr Val Gln Leu Thr Glu Ala
                85                  90                  95
Glu Arg Ala Thr His Arg Pro His Val Val His Val Asp Ala Asn Asn
            100                 105                 110
Arg Ile Val Ala Leu Gly Asp Asp Leu Ala Glu Pro Val Pro Gly Ser
        115                 120                 125
Asp Gln Lys Ser Gly Ala Leu Leu Pro
    130                 135

## Description

### Field of the Invention

The present invention relates to a method for producing L-cysteine or a related substance thereof. Specifically, the present invention relates to a bacterium suitable for the production of L-cysteine or a related substance thereof and a method for producing L-cysteine or a related substance thereof utilizing such a bacterium. L-cysteine and related substances thereof are used in the fields of drugs, cosmetics, and foods.

### Background Art

L-cysteine is conventionally obtained by extraction from keratin-containing substances such as hairs, horns, and feathers or conversion of DL-2-aminothiazoline-4-carboxylic acid as a precursor using a microbial enzyme. It is also planned to produce L-cysteine in a large scale by an immobilized enzyme method utilizing a novel enzyme. Furthermore, it is also attempted to produce L-cysteine by fermentation utilizing a microorganism.

As microorganisms having an ability to produce L-cysteine, there is known, for example, a coryneform bacterium of which intracellular serine acetyltransferase activity is increased (Patent document 1). There is also known a technique of enhancing L-cysteine-producing ability by incorporating a mutant serine acetyltransferase of which feedback inhibition by L-cysteine is attenuated (Patent documents 2 to 4).

Furthermore, as microorganisms of which L-cysteine-producing ability is enhanced by suppressing the L-cysteine decomposition system, there are known coryneform bacteria or *Escherichia* bacteria in which activity of cystathionine-β-lease (Patent document 2), tryptophanase (Patent document 5), or O-acetylserine sulfhydrylase B (Patent document 6) is decreased or deleted.

Furthermore, it is known that the ydeD gene which encodes the YdeD protein participates in secretion of the metabolic products of the cysteine pathway (Non-patent document 1). Furthermore, there are also known techniques of enhancing L-cysteine-producing ability by increasing expression of the *mar*-locus, *emr*-locus, *acr*-locus, *cmr-*locus, *mex*-gene, *bmr*-gene, or *qacA*-gene, which encodes a protein suitable for secreting a toxic substance from cells (Patent document 7), or *emrAB, emrKY, yojIH, acrEF, bcr,* or *cusA* gene (Patent document 8).

Furthermore, as an L-cysteine-producing bacterium, there is known *Escherichia coli* of which activity of the positive transcriptional regulator of the cysteine regulon encoded by the *cysB* gene is increased (Patent document 9).

Furthermore, there is known a mutant serA gene coding for 3-phosphoglycerate dehydrogenase of which feedback inhibition by serine is attenuated, and use thereof for L-cysteine production by *Escherichia coli* has been suggested (Patent documents 10 and 11).

L-aspartate-α-decarboxylase (EC 4.1.1.11) is an enzyme that catalyzes the reaction of decarboxylating L-aspartic acid thereby to generate β-alanine. It is known that L-aspartate-α-decarboxylase is generally translated as an inactive precursor protein, then subjected to processing at a specific site, and thus becomes an active protein. Although it is known that L-aspartate-α-decarboxylase suffers from competitive inhibition in the presence of L-cysteic acid (Non-patent document 2), it is not known whether it is inhibited by L-cysteine.

β-Alanine generated by the catalytic reaction of L-aspartate-α-decarboxylase is used as an intermediate for the biosynthesis of pantothenic acid. Pantothenic acid is converted into coenzyme A (CoA), and then used within organisms. Therefore, L-aspartate-α-decarboxylase can be a key enzyme of the biosynthesis of acetyl-CoA (Non-patent document 3). However, it is not known whether the biosynthesis of acetyl-CoA can be a rate-limiting factor of the production of L-cysteine.

It is known that enhancing expression of the *panD* gene encoding L-aspartate-α-decarboxylase increases the production amount of pantothenic acid (Non-patent document 4). Moreover, there is known a method for producing L-glutamic acid by using a medium containing pantothenic acid (Patent document 12).

However, any relationship between L-aspartate-α-decarboxylase or pantothenic acid and L-cysteine production is not known.

### Prior art references

### Patent documents

Patent document 1: Japanese Patent Laid-open (Kokai) No. 2002-233384
Patent document 2: Japanese Patent Laid-open No. 11-155571
Patent document 3: U.S. Patent Published Application No. 2005-0112731
Patent document 4: U.S. Patent No. 6,218,168
Patent document 5: Japanese Patent Laid-open No. 2003-169668
Patent document 6: Japanese Patent Laid-open No. 2005-245311
Patent document 7: U.S. Patent No. 5,972,663
Patent document 8: Japanese Patent Laid-open No. 2005-287333
Patent document 9: International Patent Publication WO01/27307
Patent document 10: U.S. Patent No. 5,856,148
Patent document 11: U.S. Patent Published Application No. 2005-0009162
Patent document 12: International Patent Publication WO2004/111258

### Non-patent documents

Non-patent document 1: Dassler et al., Mol. Microbiol., 36, 1101-1112 (2000)
Non-patent document 2: Williamson JM. et al., J. Biol. Chem., 1979, Aug 25;254 (16):8074-82
Non-patent document 3: Kennedy J. et al., Anal. Biochem., 2004, Apr 1;327(1):91-6
Non-patent document 4: Dusch N. et al., Appl. Environ. Microbiol., 1999 Apr;65(4):1530-9

### Disclosure of the Invention

### Object to be Achieved by the Invention

An object of the present invention is to develop a novel technique for improving L-cysteine-producing ability of bacteria and thereby provide an L-cysteine-producing bacterium and a method for producing L-cysteine, a related substance thereof, or a mixture thereof using the bacterium.

### Means for Achieving the Object

The inventor of the present invention conducted various researches in order to achieve the aforementioned object, and as a result, found that by modifying a bacterium so that L-aspartate-α-decarboxylase activity thereof was increased, L-cysteine-producing ability of the bacterium could be improved. The inventor of the present invention further found that by adding pantothenic acid to a medium used for L-cysteine production, L-cysteine-producing ability could be improved. The present invention was accomplished on the basis of the above findings.

The present invention can be thus embodied as follows.
[1] A bacterium belonging to the family *Enterobacteriaceae,* which has L-cysteine-producing ability, and is modified so that L-aspartate-α-decarboxylase activity is increased.
[2] The bacterium as mentioned above, wherein the L-aspartate-α-decarboxylase activity is increased by increasing the expression amount of *panD* gene.
[3] The bacterium as mentioned above, wherein the expression amount of the *panD* gene is increased by increasing the copy number of the *panD* gene or by modifying an expression control sequence of the *panD* gene.
[4] The bacterium as mentioned above, wherein the *panD* gene is a DNA coding for a protein of the following (A) or (B):
   (A) a protein comprising the amino acid sequence of SEQ ID NO: 8, 10, 12, 14, 16, 18, 20, 22, 24, 26, 28, 30, 32, 34, 36, 38, 40, 42, 44, 46, 48, 50, 52, 54, 56, 58, or 60;
   (B) a protein comprising the amino acid sequence of SEQ ID NO: 8, 10, 12, 14, 16, 18, 20, 22, 24, 26, 28, 30, 32, 34, 36, 38, 40, 42, 44, 46, 48, 50, 52, 54, 56, 58, or 60 but which includes one or several amino acid substitutions, deletions, insertions, or additions, and having the L-aspartate-α-decarboxylase activity.
[5] The bacterium as mentioned above, wherein the *panD* gene is a DNA of the following (a) or (b):
   (a) a DNA comprising the nucleotide sequence of SEQ ID NO: 7, 9, 11, 13, 15, 17, 19, 21, 23, 25, 27, 29, 31, 33, 35, 37, 39, 41, 43, 45, 47, 49, 51, 53, 55, 57, or 59;
   (b) a DNA hybridizable with a nucleotide sequence complementary to the nucleotide sequence of SEQ ID NO: 7, 9, 11, 13, 15, 17, 19, 21, 23, 25, 27, 29, 31, 33, 35, 37, 39, 41, 43, 45, 47, 49, 51, 53, 55, 57, or 59, or a probe that can be prepared from the nucleotide sequence under stringent conditions, and coding for a protein having the L-aspartate-α-decarboxylase activity.
[6] The bacterium as mentioned above, wherein the protein having the L-aspartate-α-decarboxylase activity has the following characteristics (1) and (2):
   (1) the glycine residue of position 24 and the serine residue of position 25 are conserved;
   (2) the lysine residue of position 9, the histidine residue of position 11, the threonine residue of position 57, and the tyrosine residue of position 58 are conserved.
[7] The bacterium as mentioned above, wherein the protein having the L-aspartate-α-decarboxylase activity further has the following characteristic (3):
   (3) the valine residue of position 15, the threonine residue of position 16, the leucine residue of position 20, the tyrosine residue of position 22, the aspartic acid residue of position 29, the glutamic acid residue of position 42, the asparagine residue of position 51, the glycine residue of position 52, the arginine residue of position 54, the isoleucine residue of position 60, the asparagine residue of position 72, the glycine residue of position 73, the alanine residue of position 74, the alanine residue of position 75, the alanine residue of position 76, the aspartic acid residue of position 83, and the isoleucine residue of position 86 are conserved.
[8] The bacterium as mentioned above, which further has one or more of the following characteristics (1) to (3):
   (1) it is modified so that the serine acetyltransferase activity is increased;
   (2) L-cysteine excretion system is enhanced;
   (3) it is modified so that the 3-phosphoglycerate dehydrogenase activity is increased.
[9] The bacterium as mentioned above, which is an *Escherichia* bacterium.
[10] The bacterium as mentioned above, which is *Escherichia coli.*
[11] The bacterium as mentioned above, which is a *Pantoea* bacterium.
[12] The bacterium as mentioned above, which is *Pantoea ananatis.*
[13] A method for producing L-cysteine, a related substance thereof, or a mixture thereof, which comprises:
   culturing the bacterium as mentioned above in a medium; and
   collecting L-cysteine, a related substance thereof, or a mixture thereof from the medium.
[14] The method as mentioned above, wherein the medium contains pantothenic acid.
[15] A method for producing L-cysteine, a related substance thereof, or a mixture thereof, which comprises:
   culturing a bacterium belonging to the family *Enterobacteriaceae,* which has L-cysteine-producing ability, in a medium containing pantothenic acid; and
   collecting L-cysteine, a related substance thereof, or a mixture thereof from the medium.
[16] The method as mentioned above, wherein the contained amount of pantothenic acid in the medium is 1 mg/L or more.
[17] The method as mentioned above, wherein the related substance of L-cysteine is L-cystine, a thiazolidine derivative, or L-methionine.

### Brief Description of the Drawings

Fig. 1 shows alignment of PanD proteins derived from various bacteria. The conserved amino acid residues are boxed.
Fig. 2 shows L-cysteine production amounts obtained with addition of pantothenic acid at various concentrations.

### Modes for Carrying out the Invention

### <1> Bacterium of the present invention

The bacterium of the present invention is a bacterium belonging to the family *Enterobacteriaceae,* which has L-cysteine-producing ability and is modified so that the L-aspartate-α-decarboxylase activity is increased.

In the present invention, L-cysteine can be L-cysteine in the free form, a salt thereof, or a mixture of them. Examples of the salt include, for example, sulfate, hydrochloride, carbonate, ammonium salt, sodium salt, and potassium salt.

In the present invention, the L-cysteine-producing ability refers to an ability of a bacterium to produce L-cysteine, a related substance thereof, or a mixture thereof and accumulate it in a medium or cells of the bacterium in such an amount that L-cysteine, the related substance thereof, or the mixture thereof can be collected from the medium or cells, when the bacterium is cultured in the medium. A bacterium having L-cysteine-producing ability means a bacterium that can produce and accumulate L-cysteine, a related substance thereof, or a mixture thereof in a medium or cells in a larger amount compared with a wild-type strain or a parent strain. A bacterium having L-cysteine-producing ability preferably means a bacterium that can produce and accumulate L-cysteine, a related substance thereof, or a mixture thereof in a medium in an amount of 0.05 g/L or more, more preferably 0.1 g/L or more, particularly preferably 0.2 g/L or more.

A portion of L-cysteine produced by the bacterium can be converted into L-cystine in the medium by formation of a disulfide bond. Further, S-sulfocysteine can be generated by the reaction of L-cysteine and thiosulfate contained in the medium (Szczepkowski T.W., Nature, vol. 182 (1958)). Furthermore, L-cysteine generated in bacterial cells can be condensed with a ketone or aldehyde, for example, pyruvic acid, which exists in the cells, to produce a thiazolidine derivative via a hemithioketal as an intermediate (refer to Japanese Patent No. 2992010). These thiazolidine derivative and hemithioketal can exist as an equilibrated mixture.

Further, L-cysteine is used as a starting material of the biosyntheses of γ-glutamylcysteine, glutathione, cystathionine, homocysteine, L-methionine, S-adenosylmethionine, and so forth. Therefore, by using a bacterium having an ability to produce any of these compounds to be produced via L-cysteine in addition to the ability to produce L-cysteine, these compounds can be produced.

Therefore, in the present invention, the L-cysteine-producing ability is not limited to an ability to accumulate only L-cysteine in a medium or cells, but also includes an ability to accumulate L-cysteine, L-cystine, such a derivative of L-cysteine as mentioned above (for example, S-sulfocysteine, a thiazolidine derivative, and a hemithioketal), such a compound to be produced via L-cysteine as mentioned above (for example, γ-glutamylcysteine, glutathione, cystathionine, homocysteine, L-methionine, and S-adenosylmethionine), or a mixture thereof in the medium or cells. In the present invention, L-cystine, such a derivative of L-cysteine as mentioned above and such a compound to be produced via L-cysteine as mentioned above are collectively referred to as a related substance of L-cysteine.

The bacterium having L-cysteine-producing ability can be a bacterium inherently having L-cysteine-producing ability, or it can be obtained by modifying a microorganism such as those described below by mutagenesis or a recombinant DNA technique so that it acquires L-cysteine-producing ability.

The bacterium used for the present invention is not particularly limited, so long as a bacterium belonging to the family *Enterobacteriaceae* such as those of the genera *Escherichia, Enterobacter, Pantoea, Klebsiella, Serratia, Erwinia, Salmonella,* and *Morganella* and having L-cysteine-producing ability is chosen. Specifically, those classified into the family *Enterobacteriaceae* according to the taxonomy used in the NCBI (National Center for Biotechnology Information) database (http://www.ncbi.nlm.nih.gov/Taxonomy/Browser/wwwtax.cgi?id 91347) can be used. As a parent strain of the family *Enterobacteriaceae* used for the modification, it is desirable to use, especially, a bacterium of the genus *Escherichia, Enterobacter, Pantoea, Erwinia, Enterobacter,* or *Klebsiella.*

Although the *Escherichia* bacteria are not particularly limited, specifically, those described in the work of Neidhardt et al. (Backmann B.J., 1996, Derivations and Genotypes of some mutant derivatives of Escherichia coli K-12, p.2460-2488, Table 1, In F.D. Neidhardt (ed.), Escherichia coli and Salmonella Cellular and Molecular Biology/Second Edition, American Society for Microbiology Press, Washington, D.C.) can be used. Examples of *Escherichia* bacteria include, for example, *Escherichia coli.* Specific examples of *Escherichia coli* include the *Escherichia coli* W3110 strain (ATCC 27325), *Escherichia coli* MG1655 strain (ATCC 47076) and so forth derived from the prototype wild-type strain, K12 strain.

These strains are available from, for example, the American Type Culture Collection (Address: 12301 Parklawn Drive, Rockville, Maryland 20852, P.O. Box 1549, Manassas, VA 20108, United States of America). That is, registration numbers are given to each of the strains, and the strains can be ordered by using these registration numbers (refer to http://www.atcc.org/). The registration numbers of the strains are listed in the catalogue of the American Type Culture Collection.

Examples of the *Enterobacter* bacteria include, for example, *Enterobacter agglomerans* and *Enterobacter aerogenes.* An example of typical strains of the genus *Enterobacter* includes *Enterobacter agglomerans* ATCC 12287 strain. Also, specifically, the strains exemplified in European Patent Publication No. 952221 can be used.

Examples of the *Pantoea* bacteria include, for example, *Pantoea ananatis, Pantoea stewartii, Pantoea agglomerans,* and *Pantoea citrea.*

Specific examples of *Pantoea ananatis* include the *Pantoea ananatis* AJ13355 strain (FERM BP-6614) and SC17 strain (FERM BP-11091). The AJ13355 strain is a strain isolated from soil in Iwata-shi, Shizuoka-ken, Japan as a strain that can proliferate in a low pH medium containing L-glutamic acid and a carbon source. The SC17 strain is a strain selected as a low phlegm-producing mutant strain from the AJ13355 strain (U.S. Patent No. 6,596,517). The *Pantoea ananatis* AJ13355 strain was deposited at the National Institute of Bioscience and Human-Technology, Agency of Industrial Science and Technology, Ministry of International Trade and Industry (currently, the National Institute of Advanced Industrial Science and Technology, International Patent Organism Depositary, Address: Tsukuba Central 6, 1-1, Higashi 1-Chome, Tsukuba-shi, Ibaraki-ken, 305-8566, Japan) on February 19, 1998 and assigned an accession number of FERM P-16644. It was then converted to an international deposit under the provisions of Budapest Treaty on January 11, 1999 and assigned an accession number of FERM BP-6614. The *Pantoea ananatis* SC17 strain was deposited at the National Institute of Advanced Industrial Science and Technology, International Patent Organism Depositary (Address: Tsukuba Central 6, 1-1, Higashi 1-Chome, Tsukuba-shi, Ibaraki-ken, 305-8566, Japan) on February 4, 2009 and assigned an accession number of FERM BP-11091. The *Pantoea ananatis* AJ13355 strain was identified as *Enterobacter agglomerans* when it was isolated, and deposited as the *Enterobacter agglomerans* AJ13355 strain. However, it was recently reclassified into *Pantoea ananatis* on the basis of nucleotide sequence analysis of 16S rRNA and so forth.

Examples of the *Erwinia* bacteria include *Erwinia amylovora* and *Erwinia carotovora,* and examples of the *Klebsiella* bacteria include *Klebsiella planticola.*

In particular, *Pantoea* bacteria, *Erwinia* bacteria, and *Enterobacter* bacteria are classified as γ-proteobacteria, and they are taxonomically very close to one another (J. Gen. Appl. Microbiol., 1997, 43, 355-361; International Journal of Systematic Bacteriology, Oct. 1997, pp.1061-1067). Therefore, some bacteria belonging to the genus *Enterobacter* were reclassified into *Pantoea agglomerans, Pantoea dispersa,* or the like, on the basis of DNA-DNA hybridization experiments etc. (International Journal of Systematic Bacteriology, July 1989, 39(3), pp.337-345). For example, some strains of *Enterobacter agglomerans* were reclassified into *Pantoea agglomerans, Pantoea ananatis,* or *Pantoea stewartii* on the basis of nucleotide sequence analysis of 16S rRNA etc. Furthermore, some bacteria belonging to the genus *Erwinia* were reclassified into *Pantoea ananas* or *Pantoea stewartii* (refer to International Journal of Systematic Bacteriology, Jan 1993;43(1), pp.162-173). In the present invention, a bacterium belonging to any of the genus *Enterobacter, Pantoea, Erwinia,* and the like can be used so long as it is a bacterium classified into the family *Enterobacteriaceae.*

Methods for imparting L-cysteine-producing ability to bacteria belonging to the family *Enterobacteriaceae* and methods for enhancing L-cysteine-producing ability of such bacteria are described below.

To impart L-cysteine-producing ability to a bacterium, methods conventionally employed in the breeding of coryneform bacteria, *Escherichia* bacteria, and so forth can be used. Such methods include acquiring an auxotrophic mutant strain, an analogue resistant strain, or a metabolic regulation mutant strain, or constructing a recombinant strain in which an L-cysteine biosynthesis enzyme is overexpressed, and so forth (see "Amino Acid Fermentation", Gakkai Shuppan Center (Ltd.), 1st Edition, published May 30, 1986, pp.77-100). In the breeding of L-cysteine-producing bacteria, one or two or more kinds of the above-described properties such as auxotrophy, analogue resistance, and metabolic regulation mutation can be imparted. Also, expression of one or two or more kinds of the L-cysteine biosynthesis enzymes can be enhanced. Furthermore, imparting such properties as auxotrophy, analogue resistance, and metabolic regulation mutation can be combined with enhancing a biosynthesis enzyme.

An auxotrophic mutant strain, L-cysteine analogue resistant strain, or metabolic regulation mutant strain having L-cysteine-producing ability can be obtained by subjecting a parent strain or wild-type strain to conventional mutagenesis, such as exposure to X-rays or UV irradiation or a treatment with a mutagen such as N-methyl-N'-nitro-N-nitrosoguanidine (MNNG) or ethyl methanesulfonate (EMS), and then selecting a strain exhibiting autotrophy, analogue resistance or a metabolic regulation mutation and having L-cysteine-producing ability from the obtained mutant strains.

Methods for imparting L-cysteine-producing ability to bacteria belonging to the family *Enterobacteriaceae* or enhancing L-cysteine-producing ability of such bacteria, and bacteria having L-cysteine-producing ability are specifically exemplified below.

### <Impartation or enhancement of L-cysteine-producing ability, and L-cysteine-producing bacteria>

L-cysteine-producing ability of a bacterium can be improved by enhancing the activity of an enzyme of the L-cysteine biosynthesis pathway or an enzyme involved in synthesis of a compound serving as a substrate in the L-cysteine biosynthesis pathway, such as L-serine. Examples of such enzymes include serine acetyltransferase (SAT) and 3-phosphoglycerate dehydrogenase (PGD). Enhancement of an enzymatic activity can be attained by enhancing expression of a gene coding for the objective enzyme, or by enhancing specific activity of the objective enzyme, as described later. Because serine acetyltransferase is inhibited by feedback inhibition by L-cysteine, the enzymatic activity of this enzyme can be enhanced by, in particular, incorporating a mutant type *cysE* gene coding for serine acetyltransferase of which feedback inhibition is attenuated or eliminated into a bacterium. Further, because 3-phosphoglycerate dehydrogenase is inhibited by feedback inhibition by serine, the enzymatic activity of this enzyme can be enhanced by, in particular, incorporating a mutant type *serA* gene coding for a mutant 3-phosphoglycerate dehydrogenase of which feedback inhibition is attenuated or eliminated into a bacterium.

As mutant SAT which is derived from *Escherichia coli* and resistant to feedback inhibition, there are specifically known the mutant SAT in which the methionine residue at position 256 is replaced with glutamate residue (Japanese Patent Laid-open No. 11-155571), the mutant SAT in which the methionine residue at position 256 is replaced with isoleucine residue (Denk, D. and Boeck, A., J. General Microbiol., 133, 515-525 (1987)), the mutant SAT having a mutation in the region from the amino acid residue at position 97 to the amino acid residue at position 273 or deletion of the C-terminus region from the amino acid residue at position 227 (International Patent Publication WO97/15673, U.S. Patent No. 6,218,168), the mutant SAT in which the amino acid sequence corresponding to positions 89 to 96 of wild-type SAT contains one or more mutations, and which is desensitized to feedback inhibition by L-cysteine (U.S. Patent Published Application No. 20050112731(A1)), the mutant SAT in which the Val residue and the Asp residue at positions 95 and 96 of wild-type SAT are replaced with Arg residue and Pro residue, respectively (name of the mutant gene: *cysE5,* U.S. Patent Published Application No. 20050112731(A1)), the mutant SAT in which the threonine residue at position 167 is replaced with an alanine residue (U.S. Patent No. 6,218,168, U.S. Patent Published Application No. 20050112731(A1)), and so forth.

The gene coding for SAT is not limited to the gene of *Escherichia coli,* and any gene coding for a protein having the SAT activity can be used. For example, an SAT isozyme of *Arabidopsis thaliana* desensitized to feedback inhibition by L-cysteine is known, and the gene encoding this SAT can also be used (FEMS Microbiol. Lett., 179, 453-459 (1999)).

Further, as a gene coding for a mutant PGD resistant to feedback inhibition by serine, the *serA5* gene (U.S. Patent No. 6,180,373) is known.

Further, L-cysteine-producing ability of a bacterium can also be improved by enhancing the L-cysteine secretion system. The L-cysteine secretion system can be enhanced by enhancing expression of a gene coding for a protein involved in secretion of L-cysteine. Examples of the protein involved in secretion of L-cysteine include the YdeD protein encoded by the ydeD gene, the YfiK protein encoded by the *yfiK* gene, and the YeaS protein encoded by the *yeaS* gene. Therefore, by enhancing expression of the ydeD gene (Dassler et al., Mol. Microbiol., 36, 1101-1112 (2000)), the *yfiK* gene (Japanese Patent Laid-open (Kokai) No. 2004-49237) or the *yeaS* gene (European Patent Laid-open No. 1016710), L-cysteine-producing ability can be enhanced. Further, by introducing a mutation into the threonine residue of position 28, the phenylalanine residue of position 137, and/or the leucine residue of position 188 of the YeaS protein, the L-cysteine secretion system is also enhanced, and L-cysteine-producing ability can be enhanced (European Patent Laid-open No. 2218729). Specifically, a mutation for replacing the threonine residue at position 28 with asparagine residue, a mutation for replacing the phenylalanine residue at position 137 with serine, glutamine, alanine, histidine, cysteine or glycine residue, and/or a mutation for replacing the leucine residue at position 188 with glutamine residue in the YeaS protein is preferred (European Patent Laid-open No. 2218729).

Further, proteins suitable for secreting a substance showing cytotoxicity include those involved in secretion of L-cysteine. Therefore, by enhancing expression of a gene coding for any one of them, the L-cysteine secretion system can be enhanced. For example, by increasing expression of the *mar* locus, *emr* locus, acr locus, *cmr* locus, *mex* gene, *bmr* gene or *qacA* gene (U.S. Patent No. 5, 972, 663), or *emrAB, emrKY, yojIH, acrEF, bcr* or *cusA* gene (Japanese Patent Laid-open (Kokai) No. 2005-287333), which encode proteins suitable for secreting a substance showing cytotoxicity from cells, L-cysteine-producing ability can be enhanced.

Furthermore, the L-cysteine-producing ability can also be improved by enhancing the sulfate/thiosulfate transport system. The sulfate/thiosulfate transport system proteins are encoded by the *cysPTWAM* cluster genes (Japanese Patent Laid-open (Kokai) No. 2005-137369, European Patent No. 1528108).

Further, L-cysteine-producing ability of a bacterium can be improved by reducing the activity of cysteine desulfhydrase, which contributes to decomposition of L-cysteine. As proteins having the cysteine desulfhydrase activity of *Escherichia coli,* cystathionine-β-lyase encoded by the *metC gene* (Japanese Patent Laid-open (Kokai) No. 11-155571, Chandra et al., Biochemistry, 21, 3064-3069 (1982)), tryptophanase encoded by the *tnaA* gene (Japanese Patent Laid-open (Kokai) No. 2003-169668, Austin Newton et al., J. Biol. Chem., 240, 1211-1218 (1965)), O-acetylserine sulfhydrylase B encoded by the *cysM* gene (Japanese Patent Laid-open (Kokai) No. 2005-245311) and MalY encoded by the *malY* gene (Japanese Patent Laid-open (Kokai) No. 2005-245311) are known. The enzymatic activity can be reduced by the methods described later.

It is preferred that the L-cysteine-producing bacterium has one or more of the following characteristics (1) to (3).
(1) The bacterium is modified so that the serine acetyltransferase activity is increased.
(2) The L-cysteine excretion system is enhanced.
(3) The bacterium is modified so that the 3-phosphoglycerate dehydrogenase activity is increased.

Specific examples of L-cysteine-producing bacteria include, but not limited to, *Escherichia* bacteria such as the *E. coli* JM15 strain transformed with multiple kinds of *cysE* gene alleles encoding serine acetyltransferase (SAT) resistant to feedback inhibition (U.S. Patent 6,218,168), *E. coli* W3110 strain in which a gene encoding a protein suitable for secretion of a cytotoxic substance is overexpressed (U.S. Patent No. 5,972,663), *E. coli* in which cysteine desulfhydrase activity is decreased (Japanese Patent Laid-open (Kokai) No. 11-155571), and *E. coli* W3110 strain in which activity of the positive transcriptional control factor of the cysteine regulon encoded by the *cysB* gene is increased (WO01/27307), *E.coli* having the plasmid pACYC-DES (Japanese Patent Laid-open (Kokai) No. 2005-137369 (U.S. Patent Published Application No. 20050124049(A1), European Patent Laid-open No. 1528108(A1))) containing the *ydeD* gene, a mutant *cysE* gene, and a mutant serA5 gene, and so forth. pACYC-DES is a plasmid obtained by inserting the above three kinds of genes into pACYC184, and expression of each of the genes is controlled by the PompA promoter.

The ability to produce compound(s) biosynthesized from L-cysteine as a starting material, such as γ-glutamylcysteine, glutathione, cystathionine, homocysteine, L-methionine, and S-adenosylmethionine, can also be imparted or enhanced by enhancing activity of an enzyme of the biosynthesis system of an objective compound, or reducing activity of an enzyme of a pathway branching from biosynthesis system of the objective compound or an enzyme that decomposes the objective compound.

For example, γ-glutamylcysteine-producing ability can be enhanced by enhancing the γ-glutamylcysteine synthetase activity, and/or reducing the glutathione synthetase activity. Further, glutathione-producing ability can be imparted or enhanced by enhancing the γ-glutamylcysteine synthetase activity and/or the glutathione synthetase activity. Further, by using a mutant γ-glutamylcysteine synthetase resistant to feedback inhibition by glutathione, the ability to produce γ-glutamylcysteine or glutathione can be enhanced. Production of glutathione is described in detail in the overview of Li et al. (Yin Li, Gongyuan Wei, Jian Chen, Appl. Microbiol. Biotechnol., 66:233-242 (2004)).

L-methionine-producing ability can be imparted or enhanced by imparting L-threonine auxotrophy or norleucine resistance (Japanese Patent Laid-open (Kokai) No. 2000-139471). In E. *coli,* the genes of the enzymes involved in the biosynthesis of L-threonine exist as the threonine operon *(thrABC),* and an L-threonine auxotrophic strain that has lost the biosynthesis ability for L-homoserine and the following compounds can be obtained by, for example, deleting the *thrBC* moiety. In a norleucine resistant strain, the S-adenosylmethionine synthetase activity is attenuated, and L-methionine-producing ability is imparted or enhanced. In *E. coli,* S-adenosylmethionine synthetase is encoded by the *metK* gene*.* L-methionine-producing ability can also be imparted or enhanced by deleting the methionine repressor or enhancing activity of an enzyme involved in the L-methionine biosynthesis, such as homoserine transsuccinylase, cystathionine γ-synthase and aspartokinase-homoserine dehydrogenase II (Japanese Patent Laid-open (Kokai) No. 2000-139471). In *E. coli,* the methionine repressor is encoded by the *metJ* gene, homoserine transsuccinylase is encoded by the *metA* gene, cystathionine γ-synthase is encoded by the *metB* gene, and aspartokinase-homoserine dehydrogenase II is encoded by the *metL* gene. Further, by using a mutant homoserine transsuccinylase resistant to feedback inhibition by L-methionine, L-methionine-producing ability can also be imparted or enhanced (Japanese Patent Laid-open (Kokai) No. 2000-139471, U.S. Patent Published Application No. 20090029424). Because L-methionine is biosynthesized via L-cysteine as an intermediate, L-methionine-producing ability can also be improved by improving L-cysteine-producing ability (Japanese Patent Laid-open (Kokai) No. 2000-139471, U.S. Patent Published Application No. 20080311632). Therefore, for imparting or enhancing L-methionine-producing ability, it is also effective to impart or enhance L-cysteine-producing ability.

Specific examples of L-methionine-producing bacteria include *E. coli* strains such as AJ11539 (NRRL B-12399), AJ11540 (NRRL B-12400), AJ11541 (NRRL B-12401), AJ11542 (NRRL B-12402) (British Patent No. 2075055), 218 strain resistant to norleucine, which is an analogue of L-methionine (VKPM B-8125, Russian Patent No. 2209248), and 73 strain (VKPM B-8126, Russian Patent No. 2215782).

Further, as an L-methionine-producing bacterium, AJ13425 (FERM P-16808, Japanese Patent Laid-open (Kokai) No. 2000-139471) derived from the *E. coli* W3110 can also be used. AJ13425 is an L-threonine auxotrophic strain in which the methionine repressor is deleted, intracellular S-adenosylmethionine synthetase activity is attenuated, and intracellular homoserine transsuccinylase activity, cystathionine γ-synthase activity, and aspartokinase-homoserine dehydrogenase II activity are enhanced. AJ13425 was deposited on May 14, 1998 at the National Institute of Bioscience and Human Technology, Agency of Industrial Science and Technology, Ministry of International Trade and Industry (currently National Institute of Advanced Industrial Science and Technology, International Patent Organism Depository, Address: Tsukuba Central 6, 1-1, Higashi 1-Chome, Tsukuba-shi, Ibaraki-ken, 305-8566, Japan), and assigned an accession number of FERM P-16808.

Because cystathionine and homocysteine are intermediates of the L-methionine biosynthesis pathway, it is effective to partially use the aforementioned methods for enhancing L-methionine-producing ability for enhancing abilities to produce these substances. As specific methods for enhancing cystathionine-producing ability, there are known a method using a methionine-auxotrophic mutant strain (Japanese Patent Application No. 2003-010654) and a method adding cysteine (or raw material for biosynthesis thereof) and/or homoserine (or raw material for biosynthesis thereof) to a fermentation medium (Japanese Patent Laid-open (Kokai) No. 2005-168422). Since homocysteine is produced by using cystathionine as a precursor, the aforementioned methods for enhancing cystathionine-producing ability are also effective for enhancing homocysteine-producing ability.

Further, the ability to produce compound(s) biosynthesized by using L-methionine as a starting material, such as S-adenosylmethionine, can also be imparted or enhanced by enhancing activity of an enzyme of the biosynthesis system of an objective compound, or reducing activity of an enzyme of a pathway branching from the biosynthesis pathway of the objective compound or an enzyme that decomposes the objective compound. For example, S-adenosylmethionine-producing ability can be imparted or enhanced by enhancing the methionine adenosyltransferase activity (European Patent Laid-open Nos. 0647712 and 1457569) or enhancing the secretion factor MdfA encoded by the *mdfA* gene (U.S. Patent No. 7,410,789).

Hereafter, methods for reducing the enzymatic activity of cysteine desulfhydrase or the like will be described. In the present invention, the expression "enzymatic activity is reduced" means that the enzymatic activity is decreased compared with that of a non-modified strain such as a wild-type strain or a parent strain, and includes a case where the activity has completely disappeared. The enzymatic activity is preferably reduced to, for example, 75% or less, 50% or less, 25% or less, or 10% or less compared with that of a non-modified strain, and the complete disappearance of the activity is particularly preferred. Depending on the type of enzyme, it can also be rather preferable not to completely eliminate the enzymatic activity.

A modification for reducing the enzymatic activity can be attained by, for example, reducing expression of a gene coding for the objective enzyme. Expression of a gene can be reduced by, for example, modifying an expression control sequence, such as promoter and SD sequence, of the gene. When an expression control sequence is modified, one or more nucleotides, more preferably two or more nucleotides, particularly preferably three or more nucleotides, of the expression control sequence are modified. Further, a part or all of the expression control sequence can be deleted. Further, expression of a gene can also be reduced by expressing an antisense RNA.

Further, a modification for reducing the enzymatic activity can also be attained by, for example, deleting a part or all of a coding region of a gene coding for an objective enzyme on a chromosome. Further, the whole gene including the sequences upstream and downstream of the gene on a chromosome can be deleted. The region to be deleted can be an N-terminus region, an internal region or a C-terminus region, so long as the enzymatic activity can be reduced. Deletion of a longer region can usually more surely inactivate a gene. Furthermore, it is preferred that reading frames upstream and downstream of the region to be deleted are not the same.

Further, a modification for reducing the enzymatic activity can also be attained by introducing a mutation for amino acid substitution (missense mutation), a stop codon (nonsense mutation), or a frame shift mutation which adds or deletes one or two nucleotides into a coding region of a gene coding for the objective enzyme on a chromosome (Journal of Biological Chemistry, 272:8611-8617 (1997); Proceedings of the National Academy of Sciences, USA, 95 5511-5515 (1998); Journal of Biological Chemistry, 266, 20833-20839 (1991)).

Further, a modification for reducing the enzymatic activity can also be attained by inserting another sequence into a coding region of a gene coding for an objective enzyme on a chromosome. Such a sequence can be inserted into any region of the gene, and insertion of a longer sequence can more surely inactivate the objective gene. It is preferred that reading frames upstream and downstream of the insertion site are not the same. The other sequence is not particularly limited so long as a sequence which decreases or deletes function of the encoded protein is chosen, and examples include a marker gene such as antibiotic resistance genes, a gene useful for L-cysteine production, and so forth.

A gene on a chromosome can be modified as described above by, for example, preparing a deletion-type gene of the gene, in which a partial sequence of the gene is deleted so that the deletion-type gene does not produce a protein that can normally function, and then transforming a bacterium with a recombinant DNA containing the deletion-type gene to cause homologous recombination between the deletion-type gene and the native gene on the chromosome, which results in substitution of the deletion-type gene for the native gene on the chromosome. The above operation can be made easier by introducing a marker gene suitable for a characteristic of the host such as auxotrophy into the recombinant DNA. The protein encoded by the deletion-type gene has a conformation different from that of the wild-type protein, even if it is even produced, and thus the function is reduced or deleted. Such gene disruption based on gene substitution utilizing homologous recombination has already been established, and examples include the method called Red-driven integration (Datsenko, K.A., and Wanner, B.L., Proc. Natl. Acad. Sci. USA, 97:6640-6645 (2000)), methods using a linear DNA such as the method of utilizing Red driven integration in combination with an excision system derived from λ phage (Cho, E.H., Gumport, R.I., Gardner, J.F., J. Bacteriol., 184:5200-5203 (2002)) (refer to WO2005/010175), methods using a plasmid containing a temperature sensitive replication origin, methods using a plasmid capable of conjugative transfer, methods utilizing a suicide vector without a replication origin in a host (U.S. Patent No. 6,303,383, Japanese Patent Laid-open (Kokai) No. 05-007491), and so forth.

Further, a modification for reducing the enzymatic activity can also be attained by, for example, mutagenesis. Examples of mutagenesis include exposure to UV irradiation or a treatment with a mutagen used for usual mutagenesis such as N-methyl-N'-nitro-N-nitrosoguanidine (MNNG), ethyl methanesulfonate (EMS) and methyl methanesulfonate (MMS).

Decrease of the enzymatic activity can be confirmed by measuring the enzymatic activity. The cysteine desulfhydrase activity can be measured by the method described in Japanese Patent Laid-open (Kokai) No. 2002-233384.

Decrease of the transcription amount of an objective gene can be confirmed by comparing the amount of mRNA transcribed from the gene with that of a non-modified strain. Examples of method for evaluating amount of mRNA include Northern hybridization, RT-PCR, and so forth (Molecular Cloning, Cold Spring Harbor Laboratory Press, Cold Spring Harbor, USA, 2001).

Decrease of the amount of an objective protein can be confirmed by Western blotting using an antibody (Molecular Cloning, Cold Spring Harbor Laboratory Press, Cold Spring Harbor, USA, 2001).

### Increase of L-aspartate-α-decarboxylase activity

The bacterium of the present invention is modified so that the L-aspartate-α-decarboxylase activity is increased. The bacterium of the present invention can be obtained by modifying a bacterium belonging to the family *Enterobacteriaceae* and having L-cysteine-producing ability such as those mentioned above so that the L-aspartate-α-decarboxylase activity is increased. Alternatively, after a bacterium is modified so that the L-aspartate-α-decarboxylase activity is increased, L-cysteine-producing ability can also be imparted or enhanced.

In the present invention, the L-aspartate-α-decarboxylase activity means the activity for catalyzing the reaction of decarboxylating L-aspartic acid thereby to generate β-alanine. In the present invention, a protein having the L-aspartate-α-decarboxylase activity is called L-aspartate-α-decarboxylase. It is expected that increase of the L-aspartate-α-decarboxylase activity increases the production amount of pantothenic acid, which is biosynthesized by using β-alanine as a metabolic intermediate.

The expression "L-aspartate-α-decarboxylase activity is increased" means that the L-aspartate-α-decarboxylase activity is increased compared with that of a non-modified strain such as a wild strain or a parent strain. Although the degree of increase of the L-aspartate-α-decarboxylase activity is not particularly limited so long as the L-aspartate-α-decarboxylase activity is increased compared with that of a non-modified strain, the L-aspartate-α-decarboxylase activity is increased to preferably 150% or more, more preferably 200% or more, still more preferably 300% or more, compared with that of a non-modified strain. Furthermore, the case where the "L-aspartate-α-decarboxylase activity is increased" includes not only a case where L-aspartate-α-decarboxylase activity is increased in a strain inherently having the L-aspartate-α-decarboxylase activity, but also a case where L-aspartate-α-decarboxylase activity is imparted to a strain not inherently having the L-aspartate-α-decarboxylase activity. Moreover, so long as the L-aspartate-α-decarboxylase activity is increased as a result, L-aspartate-α-decarboxylase that is inherently possessed by a bacterium can be attenuated and/or deleted, and then an appropriate L-aspartate-α-decarboxylase can be introduced.

A modification for increasing the L-aspartate-α-decarboxylase activity can be attained by, for example, enhancing expression of a gene coding for L-aspartate-α-decarboxylase.

Enhancement of expression of a gene can be attained by, for example, increasing the copy number of the gene.

The copy number of an objective gene can be increased by introducing the gene into a chromosome. A gene can be introduced into a chromosome by, for example, using homologous recombination. For example, a large number of copies of a gene can be introduced into a chromosome by performing homologous recombination for a sequence that exists on a chromosome in a large copy number as a target. Examples of sequence existing on a chromosome in a large copy number include, but not limited to, repetitive DNA and inverted repeats existing at the both ends of a transposon. An objective gene can be ligated on the side of an L-aspartate-α-decarboxylase gene on a chromosome in tandem or can also be introduced into an unnecessary gene on a chromosome so that the objective gene overlaps with the unnecessary gene. Such gene introduction can be attained by using a temperature sensitive vector or by using an integration vector. Alternatively, as disclosed in U.S. Patent No. 5,595,889, it is also possible to incorporate an objective gene into a transposon, and allow it to be transferred to a chromosomal DNA to introduce a large number of copies of the gene. As the transposon, for example, Mu, Tn10 and Tn5 can be used. Whether an objective gene has been introduced into a chromosome can be confirmed by Southern hybridization using a part of the objective gene as a probe.

Further, the copy number of an objective gene can also be increased by introducing a vector containing the gene into a host bacterium. For example, the copy number of an objective gene can be increased by ligating a DNA fragment containing the objective gene with a vector that functions in the host bacterium to construct an expression vector of the objective gene, and transforming the host bacterium with the expression vector. As the vector, a vector autonomously replicable in the cell of the host bacterium can be used. The vector is preferably a multicopy vector. Further, the vector preferably has a marker such as an antibiotic resistance gene for selection of the transformants. Examples of vector autonomously replicable in *Escherichia coli* cells include pUC19, pUC18, pHSG299, pHSG399, pHSG398, pACYC184, pBR322, pSTV29 (all of these are available from Takara Bio), pMW219 (NIPPON GENE), pTrc99A (Pharmacia), pPROK series vectors (Clontech), pKK233-2 (Clontech), pET series vectors (Novagen), pQE series vectors (QIAGEN), broad host spectrum vector RSF1010, and so forth.

Further, expression of a gene can be enhanced by improving the transcription efficiency of the gene. The transcription efficiency of a gene can be improved by, for example, substituting a stronger promoter for the promoter of the gene on the chromosome. The "stronger promoter" means a promoter providing improved transcription of a gene compared with the natively existing wild-type promoter. As a stronger promoter, for example, known high expression promoters, such as T7 promoter, trp promoter, lac promoter, tac promoter, and PL promoter, can be used. Further, as the stronger promoter, a highly-active type of a usual promoter can be obtained by using various reporter genes. For example, by making the -35 and -10 regions in a promoter region closer to the consensus sequence, activity of the promoter can be enhanced (International Patent Publication WO00/18935). Methods for evaluating the strength of promoters and examples of strong promoter are described in the paper of Goldstein et al. (Prokaryotic Promoters in Biotechnology, Biotechnol. Annu. Rev., 1, 105-128 (1995)), and so forth.

Further, expression of a gene can also be enhanced by improving the translation efficiency of the gene. The translation efficiency of a gene can be improved by, for example, replacing the SD sequence (also referred to as ribosome binding site (RBS)) on a chromosome with a stronger SD sequence. The "stronger SD sequence" means a SD sequence that provides improved translation of mRNA compared with the natively existing wild-type SD sequence. Examples of the stronger SD sequence include, for example, RBS of the gene 10 derived from phage T7 (Olins P.O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and translation efficiency of a gene can also be improved by modifying them.

In the present invention, sites affecting gene expression, such as promoter, SD sequence and spacer region between RBS and the start codon, are also generically called "expression control regions". An expression control region can be determined by using a promoter-search vector or gene analysis software such as GENETYX. Such expression control regions can be modified by, for example, a method using a temperature sensitive vector or the Red driven integration method (WO2005/010175).

Further, expression of an objective gene can also be enhanced by amplifying a regulator that increases expression of the gene, or deleting or attenuating a regulator that reduces expression of the gene. Examples of the regulator include, for example, those belonging to the LysR family, and so forth, and they can be found by using a database, such as EcoCyc (http://ecocyc.org/), or the like. A regulator can be modified with monitoring the increase of the transcription amount of the objective gene or the increase of the amount of the objective protein as an index.

Such methods for enhancing gene expression as mentioned above can be used independently or in an arbitrary combination.

Further, a modification that increases the L-aspartate-α-decarboxylase activity can also be attained by, for example, enhancing specific activity of the L-aspartate-α-decarboxylase. An L-aspartate-α-decarboxylase showing enhanced specific activity can be obtained by, for example, searching various bacteria. Further, a highly-active type variant of an existing L-aspartate-α-decarboxylase can also be obtained by introducing a mutation into the existing L-aspartate-α-decarboxylase. Enhancement of specific activity can be independently used, or can be used in an arbitrary combination with such methods for enhancing gene expression as mentioned above.

The method of the transformation is not particularly limited, and conventionally known methods can be used. There can be used, for example, methods of treating recipient cells with calcium chloride so as to increase permeability thereof for DNA, which has been reported for *Escherichia coli* K-12 strain (Mandel, M. and Higa, A., J. Mol. Biol., 53:159-162 (1970)), and preparing competent cells from cells which are at the growth phase, followed by transformation with DNA, which has been reported for *Bacillus subtilis* (Duncan, C.H., Wilson, G.A. and Young, F.E., Gene, 1, 153 (1977)). Alternatively, there can also be used a method of making DNA-recipient cells into protoplasts or spheroplasts, which can easily take up recombinant DNA, followed by introducing a recombinant DNA into the cells, which is known to be applicable to *Bacillus subtilis,* actinomycetes and yeasts (Chang, S. and Choen, S.N., 1979, Molec. Gen. Genet., 168:111 (1979); Bibb, M.J., Ward, J.M. and Hopwood, O.A., Nature, 274, 398 (1978); Hinnen, A., Hicks, J.B. and Fink, G.R., Proc. Natl. Acad. Sci. USA, 75, 1929 (1978)).

Increase of an objective enzymatic activity can be confirmed by measuring the enzymatic activity. The L-aspartate-α-decarboxylase activity can be measured by the method described in Non-patent document 4 (Dusch N. et al., Appl. Environ. Microbiol., 1999 Apr;65(4):1530-9).

Increase of the transcription amount of an objective gene can be confirmed by comparing amount of mRNA transcribed from the gene with that of a wild-type strain or a non-modified strain. Examples of method for evaluating amount of mRNA include Northern hybridization, RT-PCR, and so forth (Molecular Cloning, Cold spring Harbor Laboratory Press, Cold Spring Harbor (USA), 2001).

Increase of the amount of an objective protein can be confirmed by Western blotting using an antibody (Molecular Cloning, Cold Spring Harbor Laboratory Press, Cold Spring Harbor (USA), 2001).

Objects of such methods for enhancing expression of a gene or specific activity of an enzyme as mentioned above are not limited to L-aspartate-α-decarboxylase and a gene coding for it, but they can be similarly applied to arbitrary enzymes such as SAT, genes coding for them, and so forth.

L-aspartate-α-decarboxylase is generally encoded by the *panD* gene. The protein encoded by the *panD* gene is also called PanD protein. The *panD* gene of the *Escherichia coli* K12 MG1655 strain corresponds to a complementary sequence of the sequence of positions 146314 to 146694 in the genome sequence registered at the NCBI database as GenBank accession NC_000913 (VERSION NC_000913.2 GI: 49175990). The *panD* gene of the *Escherichia coli* K12 MG1655 strain is synonymous with ECK0130 or JW0127. Furthermore, the PanD protein of the *Escherichia coli* K12 MG1655 strain is registered as GenBank accession NP_414673 (version NP_414673.1 GI: 16128124, locus_tag="b0131"). The nucleotide sequences of the *panD* gene of the MG1655 strain and the amino acid sequence of the protein encoded by the gene are shown as SEQ ID NOS: 7 and 8, respectively. The nucleotide sequences of the *panD* gene of the *Pantoea ananatis* SC17 strain and the amino acid sequence of the protein encoded by the gene are shown as SEQ ID NOS: 9 and 10, respectively. The nucleotide sequences of the *panD* gene of the *Corynebacterium glutamicum* 2256 strain and the amino acid sequence of the protein encoded by the gene are shown as SEQ ID NOS: 11 and 12, respectively. The nucleotide sequences of the *panD* gene of the *Bacillus subtilis* 168 strain and the amino acid sequence of the protein encoded by the gene are shown as SEQ ID NOS: 13 and 14, respectively.

Since the nucleotide sequence of the *panD* gene can differ depending on the genus, species or strain to which the bacterium belongs, the *panD* gene to be modified so that the L-aspartate-α-decarboxylase activity is increased can be a variant of the nucleotide sequence shown as SEQ ID NO: 7, 9, 11, or 13, so long as it codes for a protein having the L-aspartate-α-decarboxylase activity. A variant of the *panD* gene can be searched for by using BLAST (http://blast.genome.jp/) or the like with referring to, for example, the nucleotide sequence of SEQ ID NO: 7, 9, 11, or 13. Furthermore, the variant of the *panD* gene includes homologues of the gene. Examples of the homologues of the *panD* gene include genes that can be amplified by PCR using a chromosome of such a microorganism as bacteria belonging to the family *Enterobacteriaceae* and coryneform bacteria as a template and synthetic oligonucleotides prepared on the basis of the nucleotide sequence of SEQ ID NO: 7, 9, 11, or 13.

Furthermore, examples of *panD* gene homologue of bacteria other than the aforementioned bacteria include *panD* genes of the following bacteria. Identities (%) of the PanD proteins encoded by the *panD* gene homologues of the bacteria relative to the PanD protein of the *Escherichia coli* K12 strain (SEQ ID NO: 8) determined by using BLAST are shown in the parentheses. The nucleotide sequences of these genes and the amino acid sequences encoded by them are shown as SEQ ID NOS: 15 to 60. In addition, the *panD* gene can be a variant of these homologues, so long as the variant codes for a protein having the L-aspartate-α-decarboxylase activity.
*Arthrobacter aurescens* ATCC BAA-1386 (54)
*Bacteroides fragilis* ATCC 25285 (44)
*Bacteroides vulgatus* ATCC 8482 (48)
*Brevibacterium lactofermentum* ATCC 13869 (43)
*Chlorobium tepidum* ATCC 49652 (47)
*Citrobacter koseri* ATCC BAA-895 (96)
*Clavibacter michiganensis* NCPPB 382 (51)
*Corynebacterium jeikeium* NCTC 11915 (48)
*Cytophaga hutchinsonii* ATCC 33406 (44)
*Enterobacter sakazakii* ATCC BAA-894 (90)
*Flavobacterium johnsoniae* ATCC 17061 (45)
*Flavobacterium psychrophilum* ATCC 49511 (44)
*Klebsiella pneumoniae* subsp. *pneumoniae* MGH78578 ATCC 700721 (89)
*Methylophilus methylotrophus* ATCC 53528 (53)
*Mycobacterium avium* subsp. *paratuberculosis* ATCC 19851 (50)
*Parabacteroides distasonis* ATCC 8503 (48)
*Porphyromonas gingivalis* ATCC 53978 (45)
*Rhodococcus* sp. (49)
*Saccharopolyspora erythraea* ATCC 11635 (51)
*Salinibacter ruber* ATCC BAA-605 (44)
*Salmonella enterica* subsp. *arizonae* serovar 62 ATCC BAA-731 (96)
*Streptomyces coelicolor* ATCC 10147 (51)
*Thermobifida fusca* ATCC 27730 (49)

The *panD* gene can be a gene coding for a protein having the amino acid sequence of the PanD protein as mentioned above, such as the amino acid sequence shown as SEQ ID NO: 8, 10, 12, 14, 16, 18, 20, 22, 24, 26, 28, 30, 32, 34, 36, 38, 40, 42, 44, 46, 48, 50, 52, 54, 56, 58, or 60, but including substitution, deletion, insertion, addition or the like of one or several amino acid residues at one or several positions, so long as it codes for a protein having the L-aspartate-α-decarboxylase activity. Although the number meant by the term "one or several" can differ depending on positions in the three-dimensional structure of the protein or types of amino acid residues, specifically, it is preferably 1 to 20, more preferably 1 to 10, still more preferably 1 to 5. The above substitution, deletion, insertion, or addition of one or several amino acid residues is a conservative mutation that maintains normal function of the protein. The conservative mutation is typically a conservative substitution. The conservative substitution is a mutation wherein substitution takes place mutually among Phe, Trp and Tyr, if the substitution site is an aromatic amino acid; among Leu, Ile and Val, if it is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of conservative substitutions include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val. The above-mentioned amino acid substitution, deletion, insertion, addition, inversion etc. can be a result of a naturally-occurring mutation (mutant or variant) due to an individual difference, a difference of species, or the like of a bacterium from which the gene is derived.

Furthermore, the gene having such a conservative mutation as mentioned above can be a gene coding for a protein showing a homology of 80% or more, preferably 90% or more, more preferably 95% or more, still more preferably 97% or more, particularly preferably 99% or more, to the total amino acid sequence of the PanD protein, such as the total sequence of the amino acid sequence of SEQ ID NO: 8, 10, 12, 14, 16, 18, 20, 22, 24, 26, 28, 30, 32, 34, 36, 38, 40, 42, 44, 46, 48, 50, 52, 54, 56, 58, or 60, and having the L-aspartate-α-decarboxylase activity. In this specification, "homology" can mean "identity".

Further, the *panD* gene can be a DNA that is able to hybridize with a probe that can be prepared from a known gene sequence, for example, a complementary sequence of the nucleotide sequence of SEQ ID NO: 7, 9, 11, 13, 15, 17, 19, 21, 23, 25, 27, 29, 31, 33, 35, 37, 39, 41, 43, 45, 47, 49, 51, 53, 55, 57, or 59, under stringent conditions, and coding for a protein having the L-aspartate-α-decarboxylase activity. The "stringent conditions" refer to conditions under which a so-called specific hybrid is formed, and a non-specific hybrid is not formed. Examples of the stringent conditions include those under which highly homologous DNAs hybridize to each other, for example, DNAs not less than 80% homologous, preferably not less than 90% homologous, more preferably not less than 95% homologous, still more preferably not less than 97% homologous, particularly preferably not less than 99% homologous, hybridize to each other, and DNAs less homologous than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

The probe can be a part of the complimentary sequence of the gene. Such a probe can be prepared by PCR using oligonucleotides prepared on the basis of a known gene sequence as primers and a DNA fragment containing the nucleotide sequence as a template. For example, when a DNA fragment having a length of about 300 bp is used as the probe, the washing conditions of the hybridization can be, for example, 50°C, 2 x SSC and 0.1% SDS.

The aforementioned explanation of the variants of genes and proteins are also similarly applied to enzymes such as serine acetyltransferase and 3-phosphoglycerate dehydrogenase, the YdeD protein, and the genes coding for them.

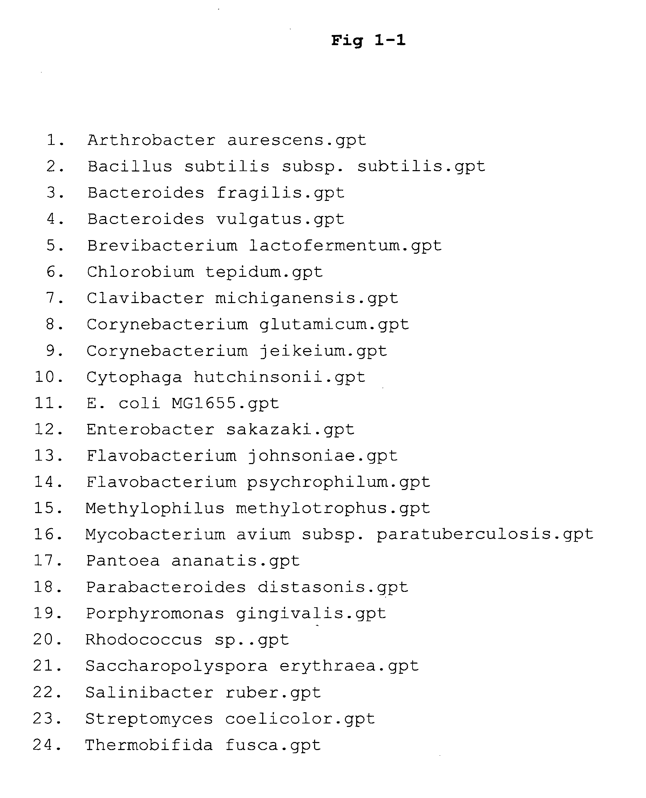

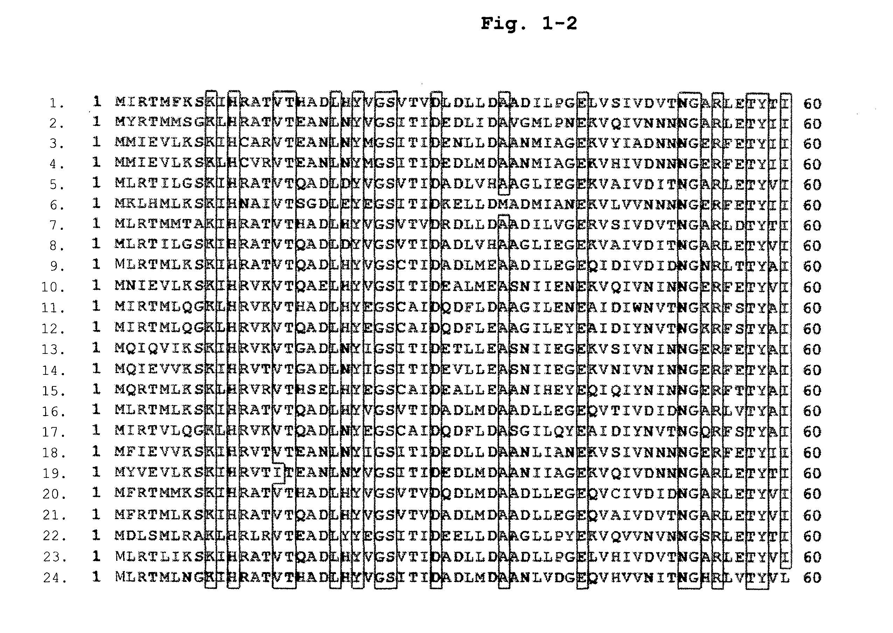

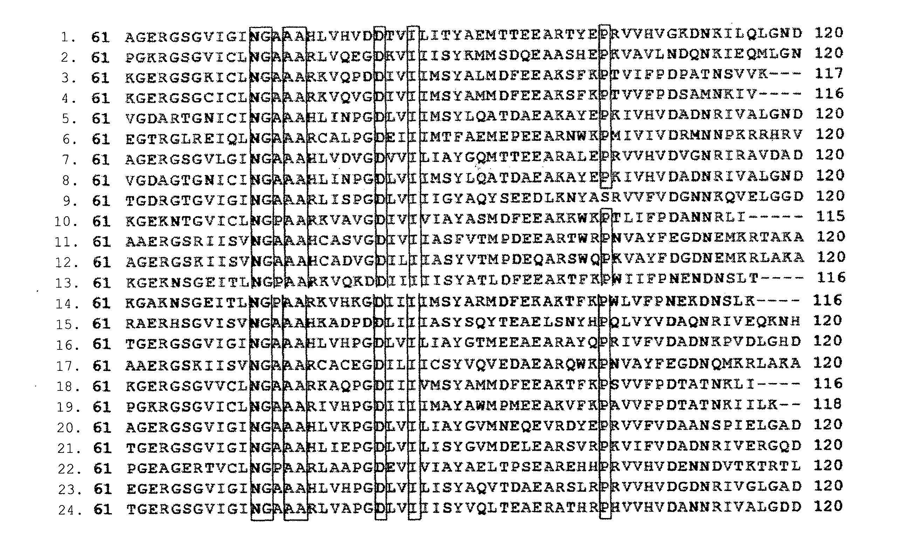

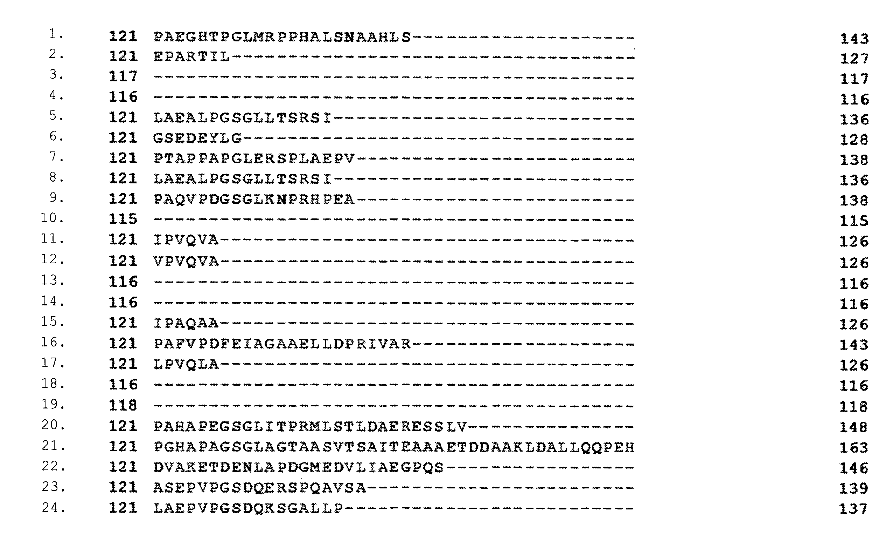

Alignment of the amino acid sequences of the PanD proteins derived from various bacteria is shown in Fig. 1. As already reported so far, the PanD proteins contain commonly conserved regions (Armando Albert et al., 1998, Nature Structural Biology, Volume 5, No. 4:289-293). Therefore, those conserved regions are preferably conserved in the PanD protein.

Specifically, it is preferred that the glycine residue of position 24 and the serine residue of position 25 are conserved in the PanD protein. The PanD protein is divided into the α subunit and β subunit between the glycine residue of position 24 and the serine residue of position 25. These amino acid residues were commonly conserved in the PanD proteins of all the species mentioned in the alignment (Fig. 1).

Furthermore, it is preferred that the lysine residue of position 9, the histidine residue of position 11, the threonine residue of position 57, and the tyrosine residue of position 58 are conserved. These amino acid residues are estimated to constitute a pocket on the basis of the three-dimensional structure, and it is thought that they contribute to the L-aspartate-α-decarboxylase activity. These amino acid residues were commonly conserved in the PanD proteins of all the species mentioned in the alignment (Fig. 1).

Furthermore, it is preferred that one or more residues selected from the valine residue of position 15, the threonine residue of position 16, the leucine residue of position 20, the tyrosine residue of position 22, the aspartic acid residue of position 29, the glutamic acid residue of position 42, the asparagine residue of position 51, the glycine residue of position 52, the arginine residue of position 54, the isoleucine residue of position 60, the asparagine residue of position 72, the glycine residue of position 73, the alanine residue of position 74, the alanine residue of position 75, the alanine residue of position 76, the aspartic acid residue of position 83, and the isoleucine residue of position 86 are conserved, and it is more preferred that all of these residues are conserved. These amino acid residues substantially commonly conserved in the PanD proteins mentioned in the alignment (Fig. 1).

Each of the aforementioned amino acid residues refers to an amino acid residue corresponding to each of the positions in SEQ ID NO: 8. That is, positions of the aforementioned amino acid residues represent relative positions, and can be changed by deletion, insertion, addition, or the like of amino acid residues. For example, the "serine residue of position 25" means the serine residue corresponding to position 25 in SEQ ID NO: 8. When one amino acid residue on the N-terminus side of position 25 is deleted, the 24th serine residue from the N-terminus (the methionine residue encoded by the start codon is also counted) shall be the "serine residue of position 25". When one amino acid residue is inserted on the N-terminus side of position 25, the 26th serine residue from the N-terminus shall be the "serine residue of position 25". Furthermore, for example, the expression "the serine residue of position 25 is conserved" in the PanD protein means that an amino acid residue corresponding to position 25 in SEQ ID NO: 8 is a serine residue in that PanD protein. The same holds for the other amino acid residues.

### <2> Method for producing L-cysteine, related substance thereof, or a mixture thereof of the present invention

By culturing the bacterium of the present invention obtained as described above in a medium and collecting L-cysteine, a related substance thereof, or a mixture thereof from the medium, these compounds can be produced. Examples of the related substance of L-cysteine include S-sulfocysteine, thiazolidine derivatives, hemithioketals corresponding to the thiazolidine derivatives, L-methionine, S-adenosylmethionine, and so forth mentioned above.

Examples of the medium to be used include ordinary media containing a carbon source, nitrogen source, sulfur source, inorganic ions, and other organic components as required.

Examples of the carbon source include saccharides such as glucose, fructose, sucrose, molasses, and starch hydrolysate, and organic acids such as fumaric acid, citric acid, and succinic acid.

Examples of the nitrogen source include inorganic ammonium salts such as ammonium sulfate, ammonium chloride and ammonium phosphate, organic nitrogen such as soybean hydrolysate, ammonia gas, and aqueous ammonia.

Examples of the sulfur source include inorganic sulfur compounds such as sulfates, sulfites, hyposulfites, thiosulfates, and sulfides.

As organic trace amount nutrients, it is desirable to add required substances such as vitamin B₁, yeast extract and so forth in appropriate amounts. Other than these, potassium phosphate, magnesium sulfate, iron ions, manganese ions, and so forth are added in small amounts, as required.

The culture is preferably performed under aerobic conditions for 30 to 90 hours. The culture temperature is preferably controlled to be at 25°C to 37°C, and pH is preferably controlled to be 5 to 8 during the culture. For pH adjustment, inorganic or organic acidic or alkaline substances, ammonia gas, and so forth can be used.

L-cysteine, a related substance thereof, or a mixture thereof can be collected from the culture broth by a combination of ion-exchange resin method, precipitation method, and other conventionally known methods.

L-cysteine obtained as described above can be used for production of L-cysteine derivatives. Examples of the L-cysteine derivatives include methylcysteine, ethylcysteine, carbocisteine, sulfocysteine, acetylcysteine, and so forth.

Furthermore, when a thiazolidine derivative of L-cysteine is accumulated in the medium, L-cysteine can be produced by collecting the thiazolidine derivative from the medium and breaking the reaction equilibrium between the thiazolidine derivative and L-cysteine so that L-cysteine is produced in a larger amount. Furthermore, when S-sulfocysteine is accumulated in the medium, it can be converted into L-cysteine by reduction using a reducing agent such as dithiothreitol.

Moreover, L-cysteine, a related substance thereof, or a mixture thereof can also be produced by adding pantothenic acid to the medium instead of modifying an L-cysteine-producing bacterium so that the L-aspartate-α-decarboxylase activity is increased. Namely, another embodiment of the method of the present invention (henceforth referred to as the second method of the present invention) is a method for producing L-cysteine, a related substance thereof, or a mixture thereof, which comprises culturing a bacterium having L-cysteine-producing ability in a medium containing pantothenic acid, and collecting L-cysteine, a related substance thereof, or a mixture thereof from the medium.

The bacterium having L-cysteine-producing ability used for the second method of the present invention may be modified or may not be modified so that the L-aspartate-α-decarboxylase activity is increased. That is, modifying an L-cysteine-producing bacterium so that the L-aspartate-α-decarboxylase activity is increased, and adding pantothenic acid to the medium can be combined.

The contained amount of pantothenic acid in the medium is preferably 1 mg/L or higher, more preferably 2 mg/L or higher, still more preferably 5 mg/L or higher, particularly preferably 10 mg/L or higher. Although the maximum contained amount of pantothenic acid is not particularly limited, it can be, for example, 10 g/L or lower, or 1 g/L or lower. Pantothenic acid can be a free acid or pantothenate salt, or can be an arbitrary mixture of them. Type of pantothenate salt is not particularly limited, and examples include calcium salt, sodium salt, and so forth.

Pantothenic acid can be contained in the medium over the entire period of the culture, or contained in the medium over only a part of the culture period. For example, if the method of the present invention comprises the stage of proliferating the L-cysteine-producing bacterium, and the stage of producing and accumulating L-cysteine, it is sufficient that pantothenic acid is contained in the medium during at least the stage of producing and accumulating L-cysteine, and pantothenic acid may be or may not be contained in the medium during the stage of proliferating the L-cysteine-producing bacterium. Further, pantothenic acid can be contained in the medium over the entire period or a part of the period of the stage of producing and accumulating L-cysteine. For example, the contained amount of pantothenic acid does not need to be within the aforementioned range over the entire period of the stage of producing and accumulating L-cysteine, that is, pantothenic acid can be contained in the medium at a contained amount within the aforementioned range in an early period of that stage, and the contained amount of pantothenic acid can be decreased with lapse of culture time. Further, pantothenic acid can be supplementarily added continuously or intermittently.

In the second method of the present invention, so long as a medium containing pantothenic acid is used, the same medium components and the same culture conditions as those of the method of using the bacterium of the present invention mentioned above can be used.

### Example

Hereafter, the present invention will be more specifically explained with reference to an example. In the following example, cysteine means L-cysteine.

### <Example>

### (1) Construction of plasmids carrying panD genes derived from C. glutamicum 2256 strain, E. coli MG1655 strain, P. ananatis SC17 strain, and B. subtilis 168 strain

In order to enhance the L-aspartate-α-decarboxylase activity, plasmids for *panD* gene expression carrying various *panD* genes were constructed by the following procedures.

By PCR using the genomic DNA of the *C. glutamicum* 2256 strain (ATCC 13869) as the template as well as primers panD(Cg)-FW (CCAAGCTTATGGCATTCCAATAGTCAGG, SEQ ID NO: 1) and panD(Cg)-RV (CGAAGCTTGGGAAAAAGACGGCTCTACC, SEQ ID NO: 2), a *panD*(Cg) gene fragment of about 900 bp containing a region from about 300 bp upstream of the *panD* gene to about 200 bp downstream of the *panD* gene was amplified. The aforementioned primers were designed so as to have a site for the restriction enzyme *Hind*III at the 5' ends. PCR was performed by using Pyrobest polymerase (Takara) in the standard reaction composition described in the protocol of the polymerase, and the reaction condition was as follows: 94°C for 5 minutes, following 30 cycles of 98°C for 5 seconds, 55°C for 5 seconds, and 72°C for 1 minute. The obtained fragment was treated with *Hind*III, and subcloned by inserting it into pSTV29 (Takara) at the *Hind*III site, and it was confirmed that the amplified sequence was correct. A fragment excised again from the plasmid with *Hind*III was inserted into pMW218 (Takara) digested with the same enzyme in the same direction as that of the *lacZ* gene on the vector thereby to obtain a plasmid pMW218-panD(Cg) (kanamycin resistance marker), in which the *panD* gene of the *C. glutamicum* 2256 strain was cloned.

Similarly, by PCR using the genomic DNA of the E. coli MG1655 strain (ATCC 47076) as the template as well as primers panD(Ec)-FW (ATGATTCGCACGATGCTGCAGG, SEQ ID NO: 3) and panD(Ec)-RV (TCAAGCAACCTGTACCGGAATCG, SEQ ID NO: 4), a *panD*(Ec) gene fragment was obtained. PCR was performed on the same conditions as mentioned above. The obtained fragment was inserted into pMW218 digested with *SmaI* in the same direction as that of the *lacZ* gene on the vector to obtain a plasmid pMW218-panD(Ec) (kanamycin resistance marker), in which the *panD* gene of the *E. coli* MG1655 strain was cloned. The cloned *panD* gene is expressed by read through from the *lac* promoter on the vector.

Similarly, by PCR using the genomic DNA of the P. *ananatis* SC17 strain (FERM BP-11091) as the template, as well as primers panD(Pa)-FW (GTAAAGGCTGCAGGTCGTATTTTTGGCTGT, SEQ ID NO: 5) and panD(Pa)-RW (GTTCTATATCGCGGTCTACTTCCTGATTCA, SEQ ID NO: 6), a *panD*(Pa) gene fragment was obtained. PCR was performed by using Pyrobest polymerase (Takara) in the standard reaction composition described in the protocol of the polymerase, and the reaction condition was as follows: 94°C for 1 minutes, following 30 cycles of 94°C for 30 seconds, 60°C for 30 seconds, and 72°C for 1 minute was repeated 30 times. The obtained fragment was inserted into pSTV29 digested with *SmaI* in the same direction as that of the *lacZ* gene on the vector to obtain a plasmid pSTV29-panD(Pa) (chroramphenicol resistance marker), in which the *panD* gene of the P. *ananatis* SC 17 strain was cloned. The cloned *panD* gene is expressed by read through from the *lac* promoter on the vector.

A plasmid pCR-panD(Bs) is a plasmid carrying a region containing the *panD* gene of the *B. subtilis* 168 strain (ATCC 23857). pCR-panD(Bs) had a structure that the region of 2350947 to 2353681 bp of the genome of the *B. subtilis* 168 strain (according to the genome database SubtiList, http://genolist.pasteur.fr/SubtiList/) was inserted into the pMW218 vector (Nippon Gene) at the *Bam*HI site by using the *Sau*3AI sites at the both ends. The *panD* gene and franking regions thereof of the *B. subtilis* 168 strain can be readily obtained by such a conventional technique as PCR, and a plasmid having a structure similar to that of pCR-panD(Bs) suitable for the object of this experiment can be constructed.

### (2) Construction of P. ananatis cysteine-producing strain SC17intE511-2

In order to investigate the effect of enhancing expression of various *panD* genes on the cysteine production, *P. ananatis* SC17intE511-2 strain having cysteine-producing ability was constructed by inserting a gene coding for an inhibition-desensitized type SAT into the chromosome of the *P. ananatis* SC17 strain according to the following procedures.

The plasmid pMIV-CysE5 (Japanese Patent Laid-open No. 2009-232844) carries the CysE5 cassette containing the gene (*cysES*) encoding a mutant SAT desensitized to feedback inhibition by L-cysteine (U.S. Patent Published Application No. 20050112731(A1)) and a chloramphenicol resistance gene. The tetracycline resistance gene excised from the plasmid pACYC184 at *Eco*88I*-Xba*I was blunt-ended, and inserted into similarly blunt-ended PvuI site of the pMIV-CysE5 thereby to construct pMT-CysE5. The CysE5 cassette was introduced into the chromosome of the *P. ananatis* wild strain, SC17 strain, by using the obtained pMT-CysE5 and a conventional mini-Mu phage system utilizing pMH10 as a helper (EP 1149911 (A)). Selection of the transformants was performed by using chloramphenicol resistance as the marker. About 50 of transformants in which the CysE5 cassette introduced into the chromosome were picked up, and cultured for cysteine production. As a result, most of the clones produced 0.2 to 0.3 g/L of cysteine. Among them, one clone produced about 0.7 g/L of cysteine, and this clone was designated as SC17intE511. The genomic DNA of SC17intE511 was extracted, and the obtained genomic DNA was introduced into SC17 by electroporation to again construct a strain in which the CysE5 cassette was introduced into the chromosome. Selection of the transformants was performed on the basis of chloramphenicol resistance as a marker. About 20 of transformants were picked up, and cultured for cysteine production. As a result, almost all of the clones produced 0.2 to 0.3 g/L of cysteine. The SAT activity of some clones among these strains was measured by a conventional method (U.S. Patent Published Application No. 20050112731(A), and a clone showing the SAT activity comparable to that of SC17intE511 as the parent strain was selected, and designated as SC17intE511-2.

### (3) Effects of enhancing expression of various panD genes and addition of pantothenic acid on cysteine production in P. ananatis cysteine-producing strain

In order to investigate the effect of enhancing expression of various *panD* genes on cysteine production, the *P. ananatis* cysteine-producing strain SC17intE511-2 was transformed with the plasmids pMW218-panD(Cg), pSTV28-panD(Pa), and pCR-panD(Bs) each, which were constructed as described above. *P. ananatis* was transformed by electroporation in a conventional manner, and selection of the transformants was performed on the LB agar medium (5 g/L of yeast extract, 10 g/L of tryptone, 10 g/L of sodium chloride, 15 g/L of agar) containing the antibiotic corresponding to the antibiotic resistance marker of each plasmid (25 mg/L of chloramphenicol or 20 mg/L of kanamycin). Moreover, as control strains, the strains transformed with empty vectors were also obtained.

Cysteine production culture was performed by using the SC17intE511-2/pMW218-panD(Cg) strain, SC17intE511-2/pMW218 strain, the SC17intE511-2/pSTV28-panD(Pa) strain, the SC17intE511-2/pSTV29 strain, the SC17intE511-2/pCR-panD(Bs) strain, and the SC17intE511-2/pCR strain obtained as described above, and the amounts of produced cysteine were compared. Cysteine production culture was also performed by using the SC17intE511-2/pSTV29 strain in a medium containing 20 mg/L of calcium pantothenate to investigate the effect of addition of pantothenic acid on cysteine production. For the culture, a cysteine production medium of the following composition containing the antibiotic corresponding to the antibiotic resistance marker of each plasmid (25 mg/L of chloramphenicol or 20 mg/L of kanamycin) was used.

### <Composition of cysteine production medium>

| | |
|---|---|
| Ammonium sulfate | 15 g/L |
| Potassium dihydrogenphosphate | 1.5 g/L |
| Magnesium sulfate heptahydrate | 1 g/L |
| Thiamine hydrochloride | 0.1 mg/L |
| Ferrous sulfate heptahydrate | 1.7 mg/L |
| Sodium molybdate dihydrate | 0.15 mg/L |
| Cobalt chloride hexahydrate | 0.7 mg/L |
| Manganese chloride tetrahydrate | 1.6 mg/L |
| Zinc sulfate heptahydrate | 0.3 mg/L |
| Copper sulfate pentahydrate | 0.25 mg/L |
| Tryptone | 0.6 g/L |
| Yeast extract | 0.3 g/L |
| Sodium chloride | 0.6 g/L |
| Calcium carbonate | 20 g/L |
| L-Histidine hydrochloride monohydrate | 135 mg/L |
| Sodium thiosulfate | 4 g/L |
| Pyridoxine hydrochloride | 2 mg/L |
| Glucose | 60 g/L |

The cysteine production culture was performed according to the following procedures. Each cysteine-producing strain was spread on the LB agar medium containing chloramphenicol or kanamycin, and precultured overnight at 34°C. The cells corresponding to about 7 cm on the plate were scraped with an inoculating loop of 10 µl size (NUNC Blue Loop), and inoculated into 2 ml of the cysteine production medium contained in a large test tube (internal diameter, 23 mm; length, 20 cm). The amounts of inoculated cells were adjusted so that the cell amounts at the time of the start of the culture should be substantially the same. The culture was performed at 32°C with shaking. When glucose in the medium was completely consumed, the culture was terminated (about 13 to 16 hours), and the amount of cysteine produced in the medium was quantified. The culture was performed in quadruplicate for each strain. Cysteine was quantified by the method described by Gaitonde, M.K. (Biochem. J., 1967 Aug., 104(2):627-33). Averages and standard deviations of the produced cysteine amounts are shown in Table 1. As shown in Table 1, it was revealed that both enhancing expression of various *panD* genes and addition of pantothenic acid increased cysteine production amount.

[Table 1]

**Table 1**

| Effect of enhancing expression of various *panD* genes and addition of pantothenic acid on cysteine production in P.*ananatis* | | |
|---|---|---|
| Vector | Cysteine (mg/L) | SD (mg/L) |
| pMW218 | 56 | 2.3 |
| pMW-panD(Cg) | 360 | 44 |
| pCR | 105 | 23 |
| pCR-panD(Bs) | 610 | 190 |
| pSTV29 | 134 | 2.4 |
| pSTV-panD(Pa) | 561 | 41 |
| pSTV29 + 20 mg/L pantothenate | 421 | 24 |

| | | |
|---|---|---|
| SD: Standard Deviation | | |

### (4) Construction of plasmid carrying inhibition-desensitized-type SAT gene, pACYC-E1

A plasmid pACYC-E1 carrying a gene coding for an inhibition-desensitized type SAT, for use in construction of a cysteine-producing bacterium, was constructed by the following procedures.

The plasmid pACYC-DE1 carries the *cysEX* gene coding for a mutant SAT desensitized to feedback inhibition by L-cysteine (U.S. Patent No. 5,972,663), and the *ydeD* gene coding for a secretion factor for cysteine and acetylserine. A cysteine-producing bacterium that can produce a marked amount of cysteine can be prepared by introducing this plasmid. pACYC-DE1 can be constructed by substantially the same method as the construction method of pACYC-DES described in EP 1528108 B1. The difference between pACYC-DE1 and pACYC-DES is that pACYC-DE1 does not carry the *serA5* gene, and that is, pACYC-DE1 corresponds to the pACYC184 vector carrying only two of the genes, the *cysEX* gene and the *ydeD* gene. In pACYC-DE1, both the *cysEX* gene and *ydeD* gene are ligated to the *ompA* promoter. This pACYC-DE1 was digested with *Mnu*I, and self-ligated to delete a region of about 330 bp inside the *ydeD* gene. The plasmid constructed as described above, which carried the *cysEX* gene, but lost the function of the *ydeD* gene, was used in the following experiments as pACYC-E1.

### (5) Effects of enhancing expression of various panD genes and addition of pantothenic acid on cysteine production in E. coli cysteine-producing strain

Whether the effects of enhancing expression of the *panD* gene and addition of pantothenic acid could also be obtained in bacteria other than *P*. *ananatis* was investigated by using an *E*. *coli* cysteine-producing strain.

As the E. *coli* cysteine-producing strain, a transformant MG1655/pACYC-E1 obtained by introducing pACYC-El (tetracycline resistance marker) mentioned above into the E. *coli* MG1655 strain by electroporation was used.

This *E*. *coli* cysteine-producing bacterium MG1655/pACYC-E1 was transformed with pMW218-panD(Cg) or pMW218-panD(Ec). The *E*. *coli* strain was transformed by electroporation in a conventional manner, and selection of the transformants was performed on the LB agar medium containing the antibiotic corresponding to the antibiotic resistance marker of each plasmid (25 mg/L of chloramphenicol or 20 mg/L of kanamycin). Moreover, as a control strain, the strain transformed with empty vector pMW218 was also obtained.

Cysteine production culture was performed by using the MG1655/pACYC-E1/pMW-panD(Cg) strain, the MG1655/pACYC-E1/pMW-panD(Ec) strain, and the MG1655/pACYC-E1/pMW-218 strain obtained as described above, and the amounts of produced cysteine were compared. Cysteine production culture was also performed by using the MG1655/pACYC-E1/pMW-218 strain in the medium containing 10 mg/L of calcium pantothenate to investigate effect of addition of pantothenic acid on cysteine production. The composition of the cysteine production medium used was the same as that mentioned above, except that the glucose concentration was 40 g/L. In the same manner as described above, the culture was continued for 19 to 22 hours until glucose in the medium was completely consumed, and the amount of cysteine produced in the medium was quantified. The culture was performed in quadruplicate for each strain. Averages and standard deviations of the produced cysteine amounts are shown in Table 2. As shown in Table 2, it was revealed that both enhancing expression of various *panD* genes and addition of pantothenic acid increased cysteine production amount also in *E*. *coli.*

[Table 2]

**Table 2**

| Effect of enhancing expression of various *panD* genes and addition of pantothenic acid on cysteine production in *E*. *coli* | | |
|---|---|---|
| Vector | Cysteine (mg/L) | SD (mg/L) |
| pMW218 | 366 | 29 |
| pMW-panD(Cg) | 742 | 47 |
| pMW-panD(Ec) | 622 | 18 |
| pMW218 | 412 | 78 |
| pMW218 + 10 mg/L pantothenate | 518 | 66 |

| | | |
|---|---|---|
| SD: Standard Deviation | | |

### (6) Investigation of added concentration of pantothenic acid

Since it was revealed above that cysteine production was increased by addition of pantothenic acid, the pantothenic acid concentration required for obtaining the effect was investigated. '

The *P*. *ananatis* SC17intE511-2/pSTV29 strain was cultured in the cysteine production medium containing calcium pantothenate at a concentration range of 10 mg/L to 100 µg/L, and cysteine production amount was measured. The composition of the cysteine production medium used was the same as that mentioned above, except that the glucose concentration was 60 g/L. The culture was performed for about 16 to 20 hours in quadruplicate for each concentration. Averages and standard deviations of the produced cysteine amounts are shown in Fig. 2. It was revealed that the cysteine production amount increased in a pantothenic acid concentration-dependent manner when 1 mg/L or more of calcium pantothenate was added.

### (7) Cysteine production with various panD gene expression-enhanced strains

Various *panD* fragments are amplified by PCR using the front 20 bp sequence and the end 20 bp sequence of nucleotide sequences of genes coding for various PanD proteins, such as the nucleotide sequences of SEQ ID NOS: 7, 9, 11, 13, 15, 17, 19, 21, 23, 25, 27, 29, 31, 33, 35, 37, 39, 41, 43, 45, 47, 49, 51, 53, 55, 57, and 59, as the forward (Fw) primer and the reverse (Rv) primer, respectively, which consists of 94°C for 5 minutes, following 30 cycles of 98°C for 5 seconds, 55°C for 5 seconds, and 72°C for 1 minute is repeated 30 times. The obtained fragment is placed under control by an appropriate promoter. For example, by introducing the obtained fragment into pSTV28 (TaKaRa) at the *Sma*I site so as to be under the control of the *lac* promoter, an expression vector of the *panD* gene is constructed. By introducing the obtained *panD* gene expression vector into a cysteine-producing bacterium, for example, *P. ananatis* SC17intE511-2, by electroporation, and performing cysteine production culture by the method described above, cysteine can be efficiently produced.

### Industrial Applicability

According to the present invention, L-cysteine-producing ability of bacteria can be improved, and L-cysteine, a related substance thereof, or a mixture thereof can be efficiently produced.

### Explanation of Sequence Listing

SEQ ID NO: 1: Primer for amplification of *C*. *glutamicum* 2256 *panD* gene
SEQ ID NO: 2: Primer for amplification of *C*. *glutamicum* 2256 *panD* gene
SEQ ID NO: 3: Primer for amplification of *E*. *coli* MG1655 *panD* gene
SEQ ID NO: 4: Primer for amplification of *E*. *coli* MG1655 *panD* gene
SEQ ID NO: 5: Primer for amplification of *P*. *ananatis* SC17 *panD* gene
SEQ ID NO: 6: Primer for amplification of *P*. *ananatis* SC17 *panD* gene
SEQ ID NO: 7: Nucleotide sequence of *E*. *coli* MG1655 *panD* gene
SEQ ID NO: 8: Amino acid sequence of *E*. *coli* MG1655 PanD protein
SEQ ID NO: 9: Nucleotide sequence of *P*. *ananatis* SC17 *panD* gene
SEQ ID NO: 10: Amino acid sequence of *P*. *ananatis* SC17 PanD protein
SEQ ID NO: 11: Nucleotide sequence of *C*. *glutamicum* 2256 *panD* gene
SEQ ID NO: 12: Amino acid sequence of *C*. *glutamicum* 2256 PanD protein
SEQ ID NO: 13: Nucleotide sequence of *B. subtilis* 168 *panD* gene
SEQ ID NO: 14: Amino acid sequence of *B. subtilis* 168 PanD protein
SEQ ID NO: 15: Nucleotide sequence of *Arthrobacter aurescens panD* gene
SEQ ID NO: 16: Amino acid sequence of *Arthrobacter aurescens* PanD protein
SEQ ID NO: 17: Nucleotide sequence of *Bacteroides fragilis panD* gene
SEQ ID NO: 18: Amino acid sequence of *Bacteroides fragilis* PanD protein
SEQ ID NO: 19: Nucleotide sequence of *Bacteroides vulgatus panD* gene
SEQ ID NO: 20: Amino acid sequence of *Bacteroides vulgatus* PanD protein
SEQ ID NO: 21: Nucleotide sequence of *Brevibacterium lactofermentum panD* gene
SEQ ID NO: 22: Amino acid sequence of *Brevibacterium lactofermentum* PanD protein
SEQ ID NO: 23: Nucleotide sequence of *Chlorobium tepidum panD* gene
SEQ ID NO: 24: Amino acid sequence of *Chlorobium tepidum* PanD protein
SEQ ID NO: 25: Nucleotide sequence of *Citrobacter koseri panD* gene
SEQ ID NO: 26: Amino acid sequence of *Citrobacter koseri* PanD protein
SEQ ID NO: 27: Nucleotide sequence of *Clavibacter michiganensis panD* gene
SEQ ID NO: 28: Amino acid sequence of *Clavibacter michiganensis* PanD protein
SEQ ID NO: 29: Nucleotide sequence of *Corynebacterium jeikeium panD* gene
SEQ ID NO: 30: Amino acid sequence of *Corynebacterium jeikeium* PanD protein
SEQ ID NO: 31: Nucleotide sequence of *Cytophaga hutchinsonii panD* gene
SEQ ID NO: 32: Amino acid sequence of *Cytophaga hutchinsonii* PanD protein
SEQ ID NO: 33: Nucleotide sequence of *Enterobacter sakazakii panD* gene
SEQ ID NO: 34: Amino acid sequence of *Enterobacter sakazakii* PanD protein
SEQ ID NO: 35: Nucleotide sequence of *Flavobacterium johnsoniae panD* gene
SEQ ID NO: 36: Amino acid sequence of *Flavobacterium johnsoniae* PanD protein
SEQ ID NO: 37: Nucleotide sequence of *Flavobacterium psychrophilum panD* gene
SEQ ID NO: 38: Amino acid sequence of *Flavobacterium psychrophilum* PanD protein
SEQ ID NO: 39: Nucleotide sequence of *Klebsiella pneumoniae panD* gene
SEQ ID NO: 40: Amino acid sequence of *Klebsiella pneumoniae* PanD protein
SEQ ID NO: 41: Nucleotide sequence of *Methylophilus methylotrophus panD* gene
SEQ ID NO: 42: Amino acid sequence of *Methylophilus methylotrophus* PanD protein
SEQ ID NO: 43: Nucleotide sequence of *Mycobacterium avium panD* gene
SEQ ID NO: 44: Amino acid sequence of *Mycobacterium avium* PanD protein
SEQ ID NO: 45: Nucleotide sequence of *Parabacteroides distasonis panD* gene
SEQ ID NO: 46: Amino acid sequence of *Parabacteroides distasonis* PanD protein
SEQ ID NO: 47: Nucleotide sequence of *Porphyromonas gingivalis panD* gene
SEQ ID NO: 48: Amino acid sequence of *Porphyromonas gingivalis* PanD protein
SEQ ID NO: 49: Nucleotide sequence of *Rhodococcus* sp. *panD* gene
SEQ ID NO: 50: Amino acid sequence of *Rhodococcus* sp. PanD protein
SEQ ID NO: 51: Nucleotide sequence of *Saccharopolyspora erythraea panD* gene
SEQ ID NO: 52: Amino acid sequence of *Saccharopolyspora erythraea* PanD protein
SEQ ID NO: 53: Nucleotide sequence of *Salinibacter ruber panD* gene
SEQ ID NO: 54: Amino acid sequence of *Salinibacter ruber* PanD protein
SEQ ID NO: 55: Nucleotide sequence of *Salmonella enterica panD* gene
SEQ ID NO: 56: Amino acid sequence of *Salmonella enterica* PanD protein
SEQ ID NO: 57: Nucleotide sequence of *Streptomyces coelicolor panD* gene
SEQ ID NO: 58: Amino acid sequence of *Streptomyces coelicolor* PanD protein
SEQ ID NO: 59: Nucleotide sequence of *Thermobifida fusca panD* gene
SEQ ID NO: 60: Amino acid sequence of *Thermobifida fusca* PanD protein

## Claims

1. A bacterium belonging to the family *Enterobacteriaceae,* which has L-cysteine-producing ability, and is modified so that L-aspartate-α-decarboxylase activity is increased.

2. The bacterium according to claim 1, wherein the L-aspartate-α-decarboxylase activity is increased by increasing the expression amount of *panD* gene.

3. The bacterium according to claim 2, wherein the expression amount of the *panD* gene is increased by increasing the copy number of the *panD* gene or by modifying an expression control sequence of the *panD* gene.

4. The bacterium according to claim 2 or 3, wherein the *panD* gene is a DNA coding for a protein of the following (A) or (B):
(A) a protein comprising the amino acid sequence of SEQ ID NO: 8, 10, 12, 14, 16, 18, 20, 22, 24, 26, 28, 30, 32, 34, 36, 38, 40, 42, 44, 46, 48, 50, 52, 54, 56, 58, or 60;
(B) a protein comprising the amino acid sequence of SEQ ID NO: 8, 10, 12, 14, 16, 18, 20, 22, 24, 26, 28, 30, 32, 34, 36, 38, 40, 42, 44, 46, 48, 50, 52, 54, 56, 58, or 60 but which includes one or several amino acid substitutions, deletions, insertions, or additions, and having the L-aspartate-α-decarboxylase activity.

5. The bacterium according to any one of claims 2 to 4, wherein the *panD* gene is a DNA of the following (a) or (b):
(a) a DNA comprising the nucleotide sequence of SEQ ID NO: 7, 9, 11, 13, 15, 17, 19, 21, 23, 25, 27, 29, 31, 33, 35, 37, 39, 41, 43, 45, 47, 49, 51, 53, 55, 57, or 59;
(b) a DNA hybridizable with a nucleotide sequence complementary to the nucleotide sequence of SEQ ID NO: 7, 9, 11, 13, 15, 17, 19, 21, 23, 25, 27, 29, 31, 33, 35, 37, 39, 41, 43, 45, 47, 49, 51, 53, 55, 57, or 59, or a probe that can be prepared from the nucleotide sequence under stringent conditions, and coding for a protein having the L-aspartate-α-decarboxylase activity.

6. The bacterium according to claim 4 or 5, wherein the protein having the L-aspartate-α-decarboxylase activity has the following characteristics (1) and (2):
(1) the glycine residue of position 24 and the serine residue of position 25 are conserved;
(2) the lysine residue of position 9, the histidine residue of position 11, the threonine residue of position 57, and the tyrosine residue of position 58 are conserved.

7. The bacterium according to claim 6, wherein the protein having the L-aspartate-α-decarboxylase activity further has the following characteristic (3): (3) the valine residue of position 15, the threonine residue of position 16, the leucine residue of position 20, the tyrosine residue of position 22, the aspartic acid residue of position 29, the glutamic acid residue of position 42, the asparagine residue of position 51, the glycine residue of position 52, the arginine residue of position 54, the isoleucine residue of position 60, the asparagine residue of position 72, the glycine residue of position 73, the alanine residue of position 74, the alanine residue of position 75, the alanine residue of position 76, the aspartic acid residue of position 83, and the isoleucine residue of position 86 are conserved.

8. The bacterium according to any one of claims 1 to 7, which further has one or more of the following characteristics (1) to (3):
(1) it is modified so that the serine acetyltransferase activity is increased;
(2) L-cysteine excretion system is enhanced;
(3) it is modified so that the 3-phosphoglycerate dehydrogenase activity is increased.

9. The bacterium according to any one of claims 1 to 8, which is an *Escherichia* bacterium.

10. The bacterium according to claim 9, which is *Escherichia coli.*

11. The bacterium according to any one of claims 1 to 8, which is a *Pantoea* bacterium.

12. The bacterium according to claim 11, which is *Pantoea ananatis.*

13. A method for producing L-cysteine, a related substance thereof, or a mixture thereof, which comprises:
culturing the bacterium according to any one of claims 1 to 12 in a medium; and
collecting L-cysteine, a related substance thereof, or a mixture thereof from the medium.

14. The method according to claim 13, wherein the medium contains pantothenic acid.

15. A method for producing L-cysteine, a related substance thereof, or a mixture thereof, which comprises:
culturing a bacterium belonging to the family *Enterobacteriaceae,* which has L-cysteine-producing ability, in a medium containing pantothenic acid; and
collecting L-cysteine, a related substance thereof, or a mixture thereof from the medium.

16. The method according to claim 14 or 15, wherein the contained amount of pantothenic acid in the medium is 1 mg/L or more.

17. The method according to any one of claims 13 to 16, wherein the related substance of L-cysteine is L-cystine, a thiazolidine derivative, or L-methionine.
